# EUROPEAN PATENT SPECIFICATION

(11) **EP 2 608 811 B1**
(45) Date of publication and mention of the grant of the patent: **28.01.2015**
(21) Application number: 11764011.0
(22) Date of filing: 26.08.2011
(51) Int. Cl.: A61K 47/48, A61P 35/00

(54) **ALUMINA NANOPARTICLE BIOCONJUGATES AND METHODS OF STIMULATING AN IMMUNE RESPONSE USING SAID BIOCONJUGATES**
ALUMINIUM-NANOPARTIKEL-BIOKONJUGATE UND VERFAHREN ZUR STIMULATION DER IMMUNREAKTION MIT DIESEN BIOKONJUGATEN
BIOCONJUGUÉS DE NANOPARTICULES D'ALUMINE ET MÉTHODES DE STIMULATION D'UNE RÉPONSE IMMUNITAIRE UTILISANT LESDITS BIOCONJUGUÉS

(30) Priority: 27.08.2010 US 377792 P
(43) Date of publication of application: 03.07.2013
(73) Proprietor: Providence Health & Services - Oregon, Portland, OR 97213 (US); The State Of Oregon Acting By And Through The State Board Of Higher Education On Behalf Of Portland State University, Portland, OR 97207-0751 (US)
(72) Inventor: HU, Hong-Ming, Happy Valley, OR 97086 (US); LI, Haiyan, Beaverton, OR 97003 (US); JIAO, Jun, Portland, OR 97221 (US)
(74) Representative: D'Arcy, Julia
(86) International application number: PCT/US2011/049398
(87) International publication number: WO 2012/027696

(56) References cited:
- WO-A2-00/25812
- US-A- 6 086 881
- SCHALLER R ET AL: "alpha-Al2O3 nanowire delivery of soluble antigen for cross- priming of cytotoxic T-cells", MICROSCOPY AND MICROANALYSIS, SPRINGER, NEW YORK, NY, US, vol. 16, no. SUPPL. 2, 1 August 2010 (2010-08-01), pages 1170-1171, XP001554993, ISSN: 1431-9276, DOI: 10.1017/S143192761006174X
- YOKOYAMA K ET AL: "Nanoscale size dependence in the conjugation of amyloid beta and ovalbumin proteins on the surface of gold colloidal particles", NANOTECHNOLOGY, IOP, BRISTOL, GB, vol. 19, no. 37, 17 September 2008 (2008-09-17), page 375101, XP020144571, ISSN: 0957-4484, DOI: 10.1088/0957-4484/19/37/375101
- HAIYAN LI ET AL: "Alpha-alumina nanoparticles induce efficient autophagy-dependent cross-presentation and potent antitumour response", NATURE NANOTECHNOLOGY, vol. 6, no. 10, 18 September 2011 (2011-09-18), pages 645-650, XP55016222, ISSN: 1748-3387, DOI: 10.1038/nnano.2011.153

## Description

### Cross-Reference to Related Applications

This application claims priority to and the benefit of U.S. Provisional Patent Application Serial No. 61/377,792, filed on August 27, 2010.

### Statement Regarding Federally Sponsored Research or Development

This invention was made with government support under grant number 1R21CA141278-01 awarded by the National Institutes of Health and grant number N00014-10-1-0082 awarded by the Office of Naval Research. The government has certain rights in the invention.

### Field

This application generally relates to alumina (Al₂O₃) nanoparticle bioconjugates and their use in immunogenic compositions such as vaccine compositions.

### Background

Cross-presentation of exogenous antigens to cytolytic T-lymphocytes (CTL) by dendritic cells (DCs) relies on the major cellular proteolysis machinery, the proteasome, to digest long polypeptides into small fragments, that can associate with cell surface molecules (MHC class I) which form the specific ligands that trigger T-cell activation. In contrast, presentation of exogenous antigens to helper T-lymphocytes (Th) by DCs primarily depends on the lysosomal pathway, where proteases inside lysosomes contribute to the digestion of internalized proteins, and digested small peptide fragments are then loaded on MHC class II molecules to activate naive Th cells.

Tumor cells process antigens that can be recognized by T lymphocytes (T cells) and activated cytolytic T lymphocytes (CTL) that constantly circulate and seek to destroy tumor cells. Therefore, methods of treating cancer using cancer immunotherapy have been proposed to generate therapeutic T cells that are reactive to tumor-associated antigens above the threshold level required to mediate regression of established tumors and prevent tumor recurrence.

Unfortunately, despite considerable effort by many investigators for many years, specific-active immunization with cancer vaccines has not been very effective in animal models or in clinical trials. The primary obstacle to the success of cancer immunotherapy has been the inability of the vaccine to induce initially a large expansion and then persistence of tumor-reactive CTL. In addition, other obstacles to currently available methods of cancer immunotherapy remain. For example, potential tumor rejection antigens are not known for most cancers, with the exception of melanoma, and the dominant tumor rejection antigens are likely tumor or patient-specific.

Schaller et al, Microscopy and Microanalysis, 16: 1170-1171 (2010) disclose conjugates of α-Al₂O₃ nanoparticles with ovalbumin. They disclose also that α-Al₂O₃ nanoparticles, as antigen carriers, are phagocytosed favorably by dendritic cells and efficiently enhance cross-presentation of ovalbumin to Thy1.1⁺ OT-I CD8⁺ T cells.

Although various strategies have been developed in recent years, including gene-modified tumor vaccines, heat shock proteins derived from tumors, DCs loaded with tumor lysates or transfected with tumor derived RNA, fusion of tumor and DCs; and exosomes secreted from tumor cells, their ability to induce a high level of tumor-specific T cells in tumor-bearing hosts has yet to be demonstrated. Therefore, new immunogenic approaches are needed for the treatment of cancer.

### Summary

In light of the foregoing, it is an object of the present teachings to provide bioconjugates that can be used to stimulate a strong immunity (such as anti-tumor or anti-pathogen immunity) via cross-priming. The bioconjugates disclosed herein can be used in immunotherapy, for example, to generate sufficient numbers of tumor- or pathogen-reactive effector/memory T cells in tumor-bearing hosts (or pathogen-infected hosts) above the threshold level required to mediate tumor (or pathogen) regression or prevent tumor (or pathogen) recurrence.

Generally, the present teachings provide bioconjugates comprising autophagosomes conjugated to alumina (e.g., α-Al₂O₃) nanoparticles, where these conjugates are capable of stimulating an immune response against a target antigen in a subject. More specifically, the alumina nanoparticles have a diameter ranging from 5 nm to 150 nm, and the autophagosomes encapsulate defective ribosomal products (DRiPs) of the target antigen.

The autophagosomes can be produced from cells that produce defective ribosomal products, for example, tumor cells or cells infected with one or more pathogens. Because cellular accumulation and secretion of DRiPs into autophagy bodies (e.g., autophagosomes) can be induced by reducing or inhibiting cellular protein degradation, autophagosomes encapsulating DRiPs can be produced by contacting cells with a proteasome inhibitor and lysosomal blocker and optionally an autophagy inducer. The autophagosomes can be produced *ex vivo* using cells (e.g., tumor cells or cells infected with one or more pathogens) obtained from the subject. The autophagosomes can be harvested from the cells to provide isolated autophagosomes. For example, the target antigen can be a tumor-associated antigen, and the autophagosome(s) can be derived from a tumor cell. In another example, the target antigen can be a pathogen-associated antigen, and the autophagosome can be derived from a cell infected with the pathogen or a vector that expresses the pathogen-associated antigen. For example, the pathogen can be a virus, in which case the autophagosome can be derived from the virus.

As described in more detail herein, alumina nanoparticles having a diameter of less than about 150 nm were shown to be capable of inducing an unexpected improvement in the cross-presentation of antigens. They also are capable of unexpectedly increasing the antitumor efficacy of tumor-derived autophagosomes containing unknown tumor-specific antigens in a limited amount. Such benefits were not observed with nanoparticles composed of other metal oxides or alumina nanoparticles having greater diameters.

Accordingly, the alumina nanoparticles of the present bioconjugates have a diameter ranging from 5 nm to 150 nm, for example, ranging from 5 nm to about 100 nm (e.g., ranging from about 10 nm to about 80 nm). Alumina nanoparticles having a diameter of less than about 150 nm can be prepared by various processes known in the art including laser pyrolysis, flame spray pyrolysis, combustion synthesis, or sol-gel approaches. In certain embodiments, bioconjugates according to the present teachings can be prepared by laser pyrolysis which is useful in the formation of particles that are highly uniform in composition, crystallinity and size.

The autophagsomes can be conjugated to the alumina nanoparticles using various coupling reactions. In some embodiments, the autophagosome(s) can be modified with a linker having a terminal hydrazine group. The alumina nanoparticle can be surface-modified with amine groups, then further modified with a linker having a terminal aldehyde (e.g., benzaldehyde) group. The hydrazine group and the aldehyde group can react to form stable hydrazone bonds that covalently attached the autophagosome(s) to the surface of the alumina nanoparticle.

Immunogenic compositions according to the present teachings can include a therapeutically effective amount of the alumina nanoparticle-autophagosome conjugate suspended in a pharmaceutically acceptable fluid carrier. In some embodiments, the immunogenic compositions can include a therapeutically effective amount of antigen-presenting cells that have been pulsed (loaded) with the alumina nanoparticle-autophagosome conjugates (referred herein as "conjugate-loaded APCs"). Such conjugate-loaded-APCs can be obtained by incubating the APCs with the alumina nanoparticle-autophagosome conjugates. In various embodiments, the APCs can be dendritic cells. The pharmaceutically acceptable fluid carrier can be selected from saline, an aqueous electrolyte solution, and a buffered aqueous solution. The immunogenic compositions further can include an anti-tumor chemotherapeutic agent, an adjuvant or other immunostimulant, or combinations thereof. The immunogenic compositions can be vaccine compositions.

The present teachings also provide the conjugates and compositions according to the claims for use in methods of stimulating an immune response against a target antigen in a subject (e.g., a human or a non-human mammal). The methods can include administering to the subject an immunogenic composition disclosed herein, thereby stimulating an immune response against one or more antigens in the subject. In some embodiments, the subject can have a tumor. In these embodiments, the subject also can be administered a therapeutically effective amount of a lymphodepletion agent prior to administering the immunogenic composition. In various embodiments, the immune response stimulated by the methods disclosed herein can be mainly cell-mediated (as opposed to antibody-mediated).

The foregoing as well as other features and advantages of the present teachings will be more fully understood from the following figures, description, examples, and claims.

### Brief Description of Drawings

It should be understood that the drawings described below are for illustration purpose. The drawings are not necessarily to scale, with emphasis generally being placed upon illustrating the principles of the present teachings.
Fig. 1a is a schematic diagram showing the structure of an embodiment of a metal oxide nanoparticle (MOₓ NP)-protein antigen conjugate according to the present teachings.
Fig. 1b is a transmission electron microscopy (TEM) image of alumina nanoparticles (α-Al₂O₃ NPs, 60 nm) before conjugation with ovalbumin (OVA) proteins. The inset shows a high-resolution TEM image of an α-Al₂O₃ NP.
Fig. 1c is a TEM image showing the surface of an α-Al₂O₃ NP (60 nm) after conjugation with OVA proteins.
Fig. 1d shows representative bright field (left), fluorescence (middle), and overlaid (right) images of dendritic cells (DCs) after incubation with fluorescein isothiocyanate (FITC)-labeled α-Al₂O₃ NP-OVA conjugates for 0.5 hour (top) and 24 hours (bottom).
Fig. 1e compares the surface expression of MHC I peptide complexes (K^{b}-SIINFEKL) on DCs without antigen (no Ag), against DCs pulsed with 10 µg/mL OVA (OVA 10 µg/mL), and DCs pulsed with α-Al₂O₃ NP (60 nm)-OVA conjugates that contain 0.1 µg/mL OVA (α-Al₂O₃ NP-OVA 0.1 µg/mL).
Fig. 2a shows the percentage of divided Thy1.1⁺ OT-I CD8⁺ T cells at 60 hours after stimulation with dendritic cells loaded with titrated amounts of OVA antigens alone as compared to those loaded with OVA antigens conjugated to α-Al₂O₃ NPs (200 nm or 60 nm), anatase TiO₂ NPs (100 nm or 25 nm), and α-Fe₂O₃ NPs (25 nm), respectively.
Fig. 2b shows INF-γ production by T cells stimulated with dendritic cells loaded with titrated amounts of OVA antigens alone as compared to those loaded with OVA antigens conjugated to α-Al₂O₃ NPs (200 nm or 60 nm), anatase TiO₂ NPs (100 nm or 25 nm), and α-Fe₂O₃ NPs (25 nm), respectively.
Fig. 2c shows IL-2 production by T cells stimulated with dendritic cells loaded with titrated amounts of OVA antigens alone as compared to those loaded with OVA antigens conjugated to α-Al₂O₃ NPs (200 nm or 60 nm), anatase TiO₂ NPs (100 nm or 25 nm), and α-Fe₂O₃ NPs (25 nm), respectively.
Fig. 2d compares the percentages of cross-primed Thy1.1⁺ OT-I CD8⁺ T cells among total cells in lymph node (LN) and in spleen (SP) measured six days after subcutaneous injection of OVA antigens alone (20 µg or 200 µg), OVA antigens (20 µg) conjugated to different metal oxide nanoparticles (specifically, α-Al₂O₃ NPs (200 nm or 60 nm), anatase TiO₂ NPs (100 nm or 25 nm), or α-Fe₂O₃ NPs (25 nm), or a mixture of OVA (20 µg)/Imject® Alum Adjuvant (Thermo Scientific) in C57BL/6 mice. * *P* < 0.05.
Fig. 3 shows the percentage of divided Thy1.1⁺ OT-I CD8⁺ T cells at 60 hours after stimulation with dendritic cells loaded with no antigens (no Ag), OVA antigens alone (0.1 µg/mL), α-Al₂O₃ NP (60 nm)-OVA (0.1 µg/mL) conjugates, or a mixture of α-Al₂O₃ NPs and OVA (0.1 µg/mL).

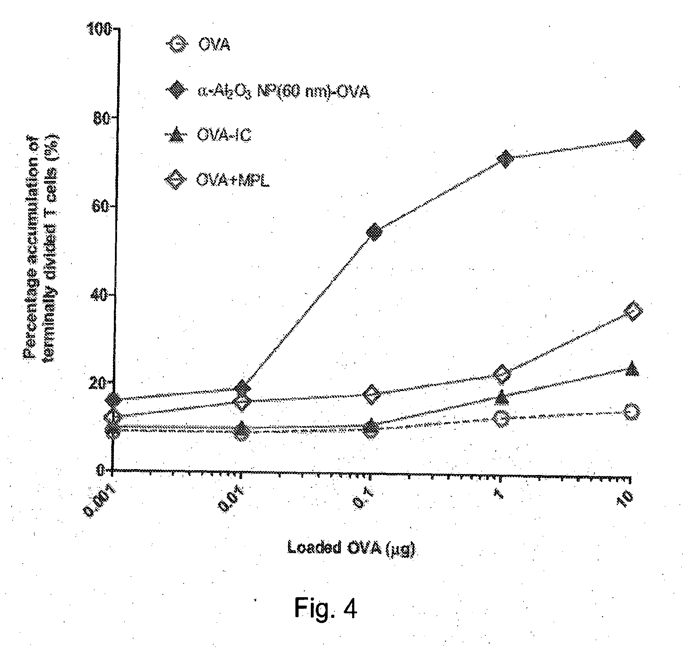

Fig. 4 shows the percentage of divided Thy1.1⁺ OT-I CD8⁺ T cells at 60 hours after stimulation with dendritic cells loaded with titrated amounts of OVA antigens alone (OVA), OVA antigens conjugated to α-Al₂O₃ NPs (α-Al₂O₃ NP (60 nm)-OVA), OVA immunocomplexes (OVA-IC), or OVA in the presence of 100 ng/mL MPL (OVA+MPL) (a TLR agonist supplied by Avanti Polar Lipids, Inc.).
Fig. 5a are TEM images of DCs loaded with α-Al₂O₃ NP-OVA conjugates, showing that the α-Al₂O₃ NP-OVA conjugates were internalized in phagosomes, lysosomes, and also autophagosomes (characterized by their double membranes).
Fig. 5b shows the effects of 3-MA and wortmannin (both autophagy inhibitors) and NH₄Cl (a lysosomal blocker) on cross-presentation of sOVA and α-Al₂O₃ NP-OVA conjugates, respectively. The percentage of divided Thy1.1⁺ OT-I CD8⁺ T cells was plotted against CFSE dilution.
Fig. 5c shows that knockdown of genes Beclin 1 or Atg 12 is required for autophagy.
Fig. 5d shows how inhibition of autophagy by knockdown of Beclin 1 or Atg 12 affected cross-presentation of sOVA and α-Al₂O₃ NP-OVA conjugates, respectively. The percentage of divided Thy1.1⁺ OT-I CD8⁺ T cells after stimulation with DCs transfected with the siRNA of Beclin 1, Atg 12, or luciferase (control) was plotted against CFSE dilution.
Fig. 5e compares LC3 II production after uptake of OVA and α-Al₂O₃ NP-OVA conjugates, respectively.
Fig. 5f shows the effects of brefeldin A (ER-Glogi blocker) on the efficiency of cross-presentation of sOVA and α-Al₂O₃ NP-OVA conjugates, respectively. DCs pulsed with NH₄Cl were used as positive control.
Fig. 5g shows that brefeldin A (ER-Glogi blocker) did not affect stimulation of naïve OT-I T cells with peptide-pulsed DCs.
Fig. 6 illustrates a proposed model of how α-Al₂O₃ NPs may divert antigens from direct endosome / lysosome degradation to indirect endosome / autophagosome / lysosome pathway.
Fig. 7a shows the frequency of OVA-specific IFN-γ producing CD8⁺ T cells in spleens of naive or tumor-bearing mice 7 days post vaccination with α-Al₂O₃ NP-OVA conjugates, α-Al₂O₃ NPs alone, or soluble OVA mixed with alum (Rehydragel®).
Fig. 7b charts the tumor size in naive or tumor-bearing mice up to 40 days post vaccination with α-Al₂O₃ NP-OVA conjugates, α-Al₂O₃ NPs alone, or soluble OVA mixed with alum (Rehydragel®).
Fig. 8a shows an SEM image of isolated autophagosome derived from lewis lung carcinoma cell lines (3LL) tumor cells. The inset shows a TEM image of an autophagosome.
Fig. 8b shows an SEM image of α-Al₂O₃ NP-autophagosome conjugates.
Fig. 8c compares the ability of DCs loaded with OVA-autophagosomes with that of DCs loaded with α-Al₂O₃ NP-OVA-autophagosomes to cross-prime naïve Thy1.1⁺ OT-I CD8⁺ T cells *in vitro.*
Fig. 8d compares the therapeutic efficacies of α-Al₂O₃ NP-autophagosome conjugates in mice bearing 3LL lung tumors with or without co-administration of anti-OX40 antibody against autophagosomes alone, or anti-OX40 antibodies alone. PBS was used as the control. * *P* < 0.05.

### Detailed Description

It is disclosed that cancer vaccines are composed of either defined antigens or undefined antigens. Defined antigens include antigenic proteins with known structures (known amino acid sequences and/or three-dimensional structures) or antigenic fragments of such proteins, where such proteins or fragments are purified and incorporated into vaccines. Examples of defined antigens include tissue-specific antigens, e.g., melanoma antigen gp100 and prostatic acid phosphatase (PAP); tumor-specific antigens, such as MAGE-3; and cancer/testis antigens, e.g., NY-ESO-1. Accordingly, a defined antigen can refer to an antigenic protein (e.g., a naturally occurring protein) in purified form. By comparison, cancer vaccines with undefined antigens include antigenic proteins that have not been fully characterized. Whole tumor cells with or without various gene modifications or fusion with DCs, or cellular components of whole cells, e.g., mRNA, secreted vesicles (exosomes), melanomasomes or other tissue specific organelles, autophagosomes (DRibbles), and apoptotic bodies including such undefined antigenic proteins can be purified and used as cancer vaccines.

Most cancer vaccine candidates require adjuvants to activate innate immunity, to overcome immune tolerance to self-antigens, and to develop a robust adoptive immune response. Currently, aluminum salts (alum) such as aluminum hydroxide, aluminum phosphate, and aluminum hydroxyphosphate compounds are the only adjuvants contained in FDA-approved vaccines for human use. However, it is well known that aluminum salts induced Th2-type rather than Th1-type immune responses. Generally, Th2-type immune responses are more effective against extracellular bacteria, parasites, and toxins, while Th1-type immune responses are more effective against intracellular pathogens (e.g., virus inside host cells). In addition, aluminum salts generally are known to be strong adjuvants for the induction of antibody responses, but very weak adjuvants for cell-mediated immune responses.

Defective ribosomal products (DRiPs) can be produced as a result of errors in protein translation or from properly translated but misfolded proteins. DRiPs can be immunogenic, and can include proteins, peptides, and/or fragments thereof. Typically, DRiPs are short-lived and are degraded by proteasomes within about 30 minutes of their synthesis.

It has been shown that when cellular protein degradation is reduced or inhibited with a proteasome inhibitor, cells accumulate and secrete DRiPs into "bleb" structures. More specifically, the inhibition of proteasome activity in the presence of lysosomal blockers has been shown to induce autophagy and secretion of short-lived proteins (SLiPs) such as DRiPs into autophagy bodies (e.g., autophagosomes) termed DRibbles. Autophagy is a cellular recycling pathway in which both cytoplasm and organelles are engulfed within double-membrane vesicles called autophagosomes, which are subsequently fused with lysosomes for degradation.

Compared to DRiPs which, even if synthesized at high levels, are rapidly destroyed before they can be delivered to antigen-presenting cells for cross-presentation, it has been shown that DRibbles, i.e., autophagosomes containing SLiPs (such as DRiPs and immunogenic fragments thereof) can act as precursors or stimulators for cross-presentation by antigen-presenting cells if they are isolated before they fuse with lysosomes or if fusion is prevented by a lysosome inhibitor. The potential use of DRibbles as immunogenic agents including as a cancer vaccine has been described in Li et al., Cancer Res., 68: 6889-6895 (2008) and International Publication No. WO 2007/016340.

Pharmaceutical agents or drugs sometimes are administered in combination with an adjuvant. An adjuvant is an agent that modifies the effect of another agent while having few if any direct effects when given by itself. An immunological adjuvant is an agent that, when used in combination with an immunogenic agent, can alter (usually enhance) a subject's immune response.

Unexpectedly, the inventors also have discovered that conjugating DRibbles (e.g., autophagosomes encapsulating DRiPs) to alumina nanoparticles having a diameter of less than 150 nm (e.g., ranging from 5 nm to about 100 nm) can provide an enhanced or increased cellular-mediated immune response compared to DRibbles alone. Without wishing to be bound to any particular theory, it is believed that the nanoparticle-autophagosome conjugates of the present teachings induce a stronger cytolytic T-cells-mediated immune response by improving the efficiency of cross-presentation and cross-priming. Compared to alum adjuvants, the inventors have found that the present nanoparticle-autophagosome conjugates are able to induce significantly more robust T-cell proliferation *in vivo*, as evidenced by a substantially higher number of Thy1.1⁺ OT-I CD8⁺ T cells in animal models. The size of the present nanoparticle-autophagosome conjugates also can be controlled as described below, unlike aluminum hydroxide which forms large precipitates (µm) when mixed with protein antigens. In addition, the inventors surprisingly have found that while conjugating autophagosomes with alumina nanoparticles having a diameter larger than 150 nm induced strong T-cell proliferation *in vitro*, the same effect was not observed *in vivo,* suggesting that conjugates using alumina nanoparticles having a diameter larger than about 150 nm are unable to cross-prime naive T cells *in vivo*.

Further, in preclinical animal tumor models of lung and breast cancers, the inventors have found that dendritic cells (DC) loaded with the nanoparticle-autophagosome conjugates of the present teachings are significantly more effective in mediating regression of established tumors compared to unconjugated (naked) autophagosomes. In addition, compared to the administration of a chemotherapeutic agent (such as an anti-OX40 antibody) alone, the chemotherapeutic agent can be made more effective in mediating regression of well-established tumors (shown in an animal model of lung cancer) when it is administered in the presence of the present nanoparticle-autophagosome conjugates.

Based on these observations, the present teachings relate to nanoparticle-autophagosome conjugates that can be used to stimulate an immune response against a target antigen, the preparation of such nanoparticle-autophagosome conjugates, and immunogenic compositions including these nanoparticle-autophagosome conjugates. In addition, the present teachings relate to the conjugates and compositions according to the claims for use in methods of stimulating an immune response against a tumor-specific DRiP (or SLiP) antigen or a pathogen-specific DRiP (or SLiP) antigen. In particular examples, such methods induce a rapid expansion of both CD4⁺ and CD8⁺ tumor-specific T cells in cancer subjects or induce a large expansion of both CD4 and CD8 pathogen-specific T cells in a subject (such as a subject infected with the pathogen).

Throughout the application, where compositions are described as having, including, or comprising specific components, or where processes are described as having, including, or comprising specific process steps, it is contemplated that compositions of the present teachings also consist essentially of, or consist of, the recited components, and that the processes of the present teachings also consist essentially of, or consist of, the recited process steps.

In the application, where an element or component is said to be included in and/or selected from a list of recited elements or components, it should be understood that the element or component can be any one of the recited elements or components, or the element or component can be selected from a group consisting of two or more of the recited elements or components.

The use of the terms "include," "includes", "including," "have," "has," or "having" should be generally understood as open-ended and non-limiting unless specifically stated otherwise.

The use of the singular herein includes the plural (and vice versa) unless specifically stated otherwise. In addition, where the use of the term "about" is before a quantitative value, the present teachings also include the specific quantitative value itself, unless specifically stated otherwise. As used herein, the term "about" or the symbol "∼" refers to a ±10% variation from the nominal value unless otherwise indicated or inferred.

It should be understood that the order of steps or order for performing certain actions is immaterial so long as the present teachings remain operable. Moreover, two or more steps or actions may be conducted simultaneously.

### Methods of Producing DRibbles

DRibbles can be produced from any type of cells that produce DRiPs or SLiPs, such as mammalian cells. Examples of such cells include, but are not limited to, tumor cells and cells infected with one or more pathogens.

Generally, cells can be contacted (incubated) with a sufficient amount of a proteasome inhibitor under conditions sufficient for producing DRibbles, such as conditions that substantially inhibit protein degradation in the cells. For example, a cell can be contacted with the proteasome inhibitor for at least 4 hours, at least 6 hours, at least 12 hours, at least 18 hours, or at least 24 hours, such as 4-24 hours, 6-24 hours, 12-24 hours, or 12-18 hours. In some embodiments, the cell optionally can be incubated with a sufficient amount of an autophagy inducer under conditions sufficient for inducing autophagy of the cell. For example, the cell can be contacted with the autophagy inducer before, during, or after the proteasome inhibitor. In certain embodiments, the cell can be contacted with the proteasome inhibitor together with agents that prevent lysosome fusion and acidification, i.e., NH₄Cl or chloroquine, for at least 4 hours (such as at least 6 hours or at least 24 hours) followed by contact with the autophagy inducer for at least 4 hours, such as at least 12 hours or at least 18 hours.

Optionally, the cell also can be contacted with other agents, such as sufficient amounts of both a proteasome inhibitor and one or more agents that decrease glycosylation of proteins (for example nucleoside translocase I inhibitors such as mureidomycin, tunicamycin, liposidomycin, or combinations thereof), under conditions sufficient to stimulate or even enhance production of DRibbles by the cell. In certain embodiments, the cell is contacted with sufficient amounts of a proteasome inhibitor (such as at least 20 nM Velcade), an autophagy inducer (such as rapamycin or HBSS) and NH₄Cl under conditions sufficient to stimulate or even enhance production of DRibbles by the cell.

DRibbles can be produced *in vivo, ex vivo,* or by a combination of both *in vivo* and *ex vivo* methods. For example, DRibbles can be produced *in vivo* by administration of a therapeutically effective amount of one or more proteasome inhibitors (alone or in combination with other agents, such as an autophagy inducer or tunicamycin, or chloroquine) to a subject, for example in an amount sufficient for producing DRibbles (such as an amount that substantially inhibits protein degradation in a tumor cell or a cell infected with a pathogen). The amount of proteasome inhibitor administered preferably is a dose that does not significantly induce apoptosis of a cell, such as a tumor cell. In another example, DRibbles are produced *ex vivo* by incubating a sufficient amount of one or more proteasome inhibitors (alone or in combination with other agents, such as an autophagy inducer, tunicamycin, or chloroquine) with cells growing in culture, for example in an amount sufficient for producing DRibbles (such as an amount that substantially inhibits protein degradation in the cell).

The DRibbles produced by the methods described herein can be separated (harvested) from the cells and then collected. It is disclosed that separation of DRibbles from cell and cell debris may result in a population of isolated DRibbles, such as a population that is at least 50% pure (i.e., containing 50% DRibbles by weight of total cellular materials), for example, at least 90% pure, at least 95% pure, or at least 99% pure. As used herein, an "isolated" biological component has been substantially separated or purified away from other biological components of the organism (or cell) in which the component naturally occurs. As such, "isolated" DRibbles are those which have been substantially separated or purified away from other biological components, such as whole cells or large cell debris.

One particular exemplary method of producing a purified population of DRibbles *ex vivo* includes contacting a cell with a sufficient amount of a composition that includes a proteasome inhibitor under conditions sufficient to substantially inhibit protein degradation in the cell, such as an incubation of about 6-24 hours. The cells are subsequently incubated under conditions sufficient to induce autophagy in the cell, such as an incubation of about 6-24 hours with an autophagy inducer. The resulting cells and DRibbles are centrifuged under conditions that pellet the cells but not the DRibbles. The supernatant containing the DRibbles is centrifuged under conditions sufficient to pellet the DRibbles. The resulting pellet containing a purified population of DRibbles is collected. The DRibbles can be used for conjugation immediately, or cryopreserved for later use.

### DRibbles from Tumor Cells

DRibbles may be produced by mammalian tumor cells, such as cells from a hematological or solid tumor. The cell may be a human cancer cell. DRibbles may be generated *ex vivo* from a tumor cell obtained from the subject to be treated. Tumor cells can be obtained from a subject using methods known in the art, such as from a surgically extracted tumor, or from a biopsy sample (such as a needle aspirate of the tumor). For example, tumor cells can be obtained from the subject and grown as a primary culture using tissue culture methods known in the art. Ideally, primary tumor cells can grow and expand well in culture, or the tumor sample obtained is large such that sufficient numbers of cells (such as at least 1 million cells) can be used for DRibble generation. Examples of tumors that grow well in culture, or tend to produce large-sized tumors having numerous cells such that primary cultures can be used, include leukemia, lymphoma, melanomas, lung cancers, ovarian cancer, gastric and colon carcinoma, and renal cell carcinomas.

However, some primary tumor cells are difficult to grow or expand in culture. For those tumor cells, an established cell line for the same type of tumor as is present in the subject can be used, or DRibbles can be produced *in vivo* by inducing autophagy of tumor cells with chemotherapeutics, radiation, or other interventions. Examples of tumors, whose cells are difficult to grow in culture, include breast and prostate cancers.

### DRibbles from Cancer Cell Lines

For example, if the subject has a breast cancer with cells that do not grow well in culture, DRibbles can be generated from a breast cancer cell line established from another subject. Examples of such cell lines are known in the art, such as MDA-MB-231 for breast cancer and PC3 and LNCap for prostate cancer.

### DRibbles from Infected Cells

DRibbles may be produced *ex vivo* from a mammalian cell infected with one or more pathogens, or a cell infected with a vector (such as a plasmid or viral vector) that includes a nucleic acid molecule encoding a pathogenic antigen. Exemplary pathogens include viruses, bacteria, fungi, protozoa, and combinations thereof. For example, viruses include positive-strand RNA viruses and negative-strand RNA viruses. Exemplary positive-strand RNA viruses include, but are not limited to, Picornaviruses (such as Aphthoviridae, e.g., foot-and-mouth-disease virus (FMDV)), Cardioviridae; Enteroviridae (e.g., Coxsackie viruses, Echoviruses, Enteroviruses, and Polio viruses); Rhinoviridae (Rhinoviruses); Hepataviridae (Hepatitis A viruses); Togaviruses (examples of which include rubella; alphaviruses (such as Western equine encephalitis virus, Eastern equine encephalitis virus, and Venezuelan equine encephalitis virus)); Flaviviruses (examples of which include Dengue virus, West Nile virus, and Japanese encephalitis virus); and Coronaviruses (examples of which include SARS coronaviruses, such as the Urbani strain). Exemplary negative-strand RNA viruses include, but are not limited to, Orthomyxyoviruses (such as the influenza virus), Rhabdoviruses (such as Rabies virus), and Paramyxoviruses (examples of which include measles virus and respiratory syncytial virus).

Viruses also include DNA viruses. DNA viruses include, but are not limited to, Hepatitis B viruses, Herpesviruses such as Varicella-zoster virus, for example, the Oka strain; cytomegalovirus; and Herpes simplex virus (HSV) types 1 and 2.

Another group of viruses includes retroviruses. Examples of retroviruses include, but are not limited to, human immunodeficiency virus type 1 (HIV-1), such as subtype C, HIV-2; equine infectious anemia virus; feline immunodeficiency virus (FIV); feline leukemia viruses (FeLV); simian immunodeficiency virus (SIV); and avian sarcoma virus.

Another type of pathogen is bacteria. Bacteria can be classified as gram-negative or gram-positive. Exemplary gram-negative bacteria include, but are not limited to, Escherichia coli (K-12 and O157:H7 and Shigella dysenteriae. Exemplary gram-positive bacteria include, but are not limited to, Bacillus anthracis, Staphylococcus aureus, pneumococcus, gonococcus, Streptococcal meningitis, and Mycobacterium tuberculosis.

Protozoa and fungi are additional types of pathogens. Exemplary protozoa include, but are not limited to, Plasmodium, Leishmania, Acanthamoeba, Giardia, Entamoeba, Cryptosporidium, Isospora, Balantidium, Trichomonas, Trypanosoma, Naegleria, and Toxoplasma. Exemplary fungi include, but are not limited to, Candida albicans, Cryptococcus, Coccidiodes immitis, and Blastomyces dermatitidis.

To produce infected-cell-derived DRibbles *ex vivo,* pathogens can be used to infect cells (for example *in vitro*), and the infected cells can be used to produce DRibbles. The particular cell type infected can depend on the pathogen used. Methods of infecting cells with particular pathogens are known. Generally, methods include incubating a cell capable of infection by the pathogen, under conditions sufficient for the pathogen to infect the cell. In some embodiments, pathogens are incubated with cells at 37°C in culture medium for at least 30 minutes, such as at least 60 minutes. Pathogens that did not infect the cells can be removed by washing the cells. Although particular examples are provided herein, one skilled in the art will appreciate that other combinations of cells and pathogens can be used.

Mycobacterium tuberculosis bacteria can be used to infect macrophages (such as macrophages obtained from PBMCs), for example using the methods described in the literature (*see e.g.,* Li et al., Infect. Immun. 70:6223-30, 2002). Plasmodium protozoa (such Plasmodium falciparum) can be used to infect erythrocytes or hepatocytes. Histoplasma or Cryptococcus fungi can be used to infect megakaryocytes. HIV can be used to infect epithelial cells or lymphocytes.

The infected cells then can be incubated with a sufficient amount of proteasome inhibitor (alone or in the presence of other agents, such as an autophagy inducers, tunicamycin, or NH₄Cl), for example under conditions sufficient to substantially inhibit protein degradation, thereby permitting the cell to generate DRibbles.

### Proteasome Inhibitors

Proteasome inhibitors are agents that can reduce and, in some cases, eliminate the proteasome-mediated catabolic pathway that degrades intracellular proteins, such as ubiquitinated proteins. In particular examples, such proteasome inhibitors block the MHC class I antigen processing pathway. Proteasome inhibitors can be reversible (such as MG132) or irreversible (such as lactacystin and epoxomicin).

Particular examples of proteasome inhibitors are peptidyl boronic acid ester and acid compounds. Exemplary proteasome inhibitors include, but are not limited to: carbobenzyloxy-L-leucyl-L-leucyl-L-leucinal (MG-132), carbobenzyloxy-L-leucyl-L-leucyl-L-norvalinal (MG-115), epoxomicin, N-benzyloxycarbonyl-L-leucyl-L-leucyl-L-leucyl boronic acid (MG-262), N-benzyloxycarbonyl-Ile-Glu(O-t-butyl)-Ala-leucinal (PSI; and its epoxide), N-Acetyl-Leu-Leu-norleucinal (MG-101, ALLN, or calpain inhibitor I), MLN519, N-pyrazinecarbonyl-L-phenylalanine-L-leucineboronic acid (bortezomib, PS-341, or VELCADE), lactacystin (Calbiochem-Novobiochem Co., La Jolla, Calif.), PS-273, N-acetyl-Leu-Leu-Met (ALLM or calpain inhibitor II), N-tosyl-Lys chloromethyl ketone (TLCK), N-tosyl-Phe chloromethyl ketone (TPCK), pyrrolidine dithiocarbamate (PDTC), [2S,3S]-trans-epoxysuccinyl-L-leucylamido-3-methylbutane ethyl ester (EST), and pentoxyfilline (PTX).

Proteasome inhibitors can be used at concentrations and under conditions that substantially inhibit protein degradation in the cells, such as inhibit such degradation by at least 90%, thereby permitting formation of DRibbles by the cells. In particular examples, proteasome inhibitors are used at a sub-lethal dose, such as concentrations that do not significantly induce apoptosis of cells, such as do not induce apoptosis in more than 10% of the cells, for example, as compared to an amount of apoptosis in the absence of the proteasome inhibitor. This is in contrast to amounts of proteasome inhibitors currently administered to subjects having a tumor, wherein the proteasome inhibitor is administered to cause apoptosis of the tumor cells. According to the present teachings, the concentrations of proteasome inhibitors used to generate DRibbles are lower than these amounts, such that the tumor cells produce DRibbles, and can be subsequently killed by tumor-specific T cells.

It is disclosed that DRibbles can be generated by contacting cells with one or more proteasome inhibitors for at least 6 hours (such as at least 8 hours, at least 12 hours, or even at least 16 hours, for example overnight treatment). Appropriate concentrations and incubation conditions can be determined using known methods by those skilled in the art.

### Autophagy Inducers

Autophagy can be induced by nutrient deprivation of cells (for example by incubation in HBSS), incubation of cells under ischemic conditions, subjection of cells to stimuli that result in organelle proliferation, and/or incubation of cells with agents that inhibit proteasome function for prolonged periods of time (for example, by incubation of cells in 20-1000 nM VELCADE), tamoxifen, rapamycin (such as 1 nM-100 nM), vinblastine (such as 5-100 mg/kg body weight vinblastine sulfate, for example 50 mg/kg body weight vinblastine sulfate), and IFN-γ (such as 10-1000 U/ml). Autophagy inducers can be used at concentrations and under conditions that substantially induce autophagy in the cells, thereby permitting formation of autophagy bodies by the cells. The rate of autophagy increases when a cell is contacted with an autophagy inducer. Methods of determining whether autophagy has been induced are known in the art.

It is disclosed that DRibbles can be generated by contacting cells with one or more proteasome inhibitors for at least 6 hours (such as at least 48 hours), followed by contacting the cells with one or more autophagy inducers for at least 6 hours (such as at least 18 hours). Appropriate concentrations and incubation conditions can be determined using known methods by those skilled in the art.

### Harvesting DRibbles

Harvesting DRibbles can include separating DRibbles from the cells, for example by collecting secreted DRibbles, by lysing cells and collecting intracellular DRibbles, or combinations thereof.

For example, DRibbles secreted by the cell into the cell culture medium can be isolated. It is disclosed that centrifugation can be used. For example, cells and the culture medium can be centrifuged under conditions sufficient to pellet whole cells and large cell debris, but not the DRibbles (for example by low-speed centrifugation). The pellet of whole cells can be used to obtain intracellular DRibbles. The resulting supernatant containing DRibbles can be centrifuged under conditions sufficient to pellet the DRibbles (such as high-speed centrifugation).

The DRibble pellet obtained using low- and high-speed centrifugation described above can be purified further by ultracentrifugation in a Percoll colloidal density gradient. For example, the DRibble pellet can be layered on top of a discontinuous gradient of 21 ml of 33% Percoll in PBS on top of 7 ml of 22.5% Nycodenz in PBS (1.127 g/ml), and centrifuged for 30 minutes at 72,000 g in a SW28 rotor. Autophagy bodies (including autophagosomes, e.g., DRibbles) will be banded at the lower interface, while apoptotic bodies or released mitochondria will be pelleted on the bottom of the tube. Other light membranes, such as endoplasmic reticulum (ER) and debris of plasma membrane, will be banded in the upper interface.

Intracellular DRibbles also can be obtained, for example by lysing the cell and substantially separating the DRibbles from other cell debris.

As used herein, "purified" does not require absolute purity; rather, it is intended as a relative term. Thus, for example, a purified cell is one in which the cell is more pure than the cell in its natural environment, such as within an organism. Similarly, a purified DRibble is one in which the DRibble is more pure than the DRibble in its natural environment, such as within a cell or culture medium. In particular examples, purified populations of DRibbles refers to populations of DRibbles that are at least 75% pure, at least 80% pure, at least 90% pure, at least 95% pure, at least 97% pure, at least 98% pure, or at least 99% pure. In one example, a substantially purified population of DRibbles is composed of at least 95% DRibbles, that is, the population of DRibbles includes less than about 5% of whole cells or large cell debris. The purity of a DRibble population can be measured based on size or by ability to stimulate a particular immune response (for example, as measured by an ELISA assay), as compared to a control.

In particular embodiments, the resulting substantially isolated population of DRibbles are at least 70% pure (i.e., containing 70% DRibbles by weight of the total amount of cellular materials), such as at least 80% pure, at least 90% pure, at least 95% pure, or even at least 99% pure. DRibbles can be used for conjugation immediately, or cryopreserved (for example at a temperature between about -20°C and about -80°C) until use. In some embodiments, isolated DRibbles can be preserved in the presence of DMSO.

### Isolated DRibbles

As used herein, isolated DRibbles or isolated autophagosomes can refer to an isolated population of DRibbles or autophagosomes that has been substantially purified, such as at least 70% pure, at least 80% pure, at least 90% pure, or even at least 95% pure. It is disclosed that an isolated population of DRibbles can be frozen, for example in the presence of at least 10% DMSO.

### Alumina Nanoparticles

Alumina nanoparticles that are suitable for use as part of the present nanoparticle-antigen conjugates have a diameter ranging from 5 nm to 150 nm. As shown in Example 4, alumina nanoparticles having a diameter of about 200 nm were shown to be unable to stimulate a T cell-mediated immune response *in vivo.* Accordingly, alumina nanoparticles for use in the present teachings have a diameter less than 150 nm, for example, having a diameter ranging from 5 nm to about 120 nm, from 5 nm to about 100 nm, from about 10 nm to about 100 nm, from about 10 nm to about 90 nm, from about 10 nm to about 80 nm, from about 10 nm to about 75 nm, from about 10 nm to about 70 nm, from about 10 nm to about 60 nm, from about 10 nm to about 50 nm, from about 10 nm to about 40 nm, or from about 10 nm to about 30 nm. In certain embodiments, the alumina nanoparticles of the present conjugates have a diameter of about 75 nm. In certain embodiments, the alumina nanoparticles of the present conjugates have a diameter of about 60 nm.

In terms of composition, as demonstrated by the data in Examples 3 and 4 below, nanoparticles of metal oxides other than alumina, regardless of their sizes, were shown to be inefficient in inducing T cell proliferation either in lymph node or in spleen *in vivo*. In addition, unlike the present alumina bioconjugates, these other metal oxide bioconjugates also were unable to stimulate significant production of cytokines.

Alumina nanoparticles suitable for the present bioconjugates can be synthesized by various methods known in the art such as chemical precipitation/coprecipitation, hydrothermal synthesis, physical vapor deposition, chemical vapor deposition, flame spray synthesis, combustion, laser pyrolysis, sol-gel synthesis, microemulsions, sonochemical synthesis, and so forth. In preferred embodiments, the alumina nanoparticles are synthesized by a method that allows control over particle size and can provide alumina nanoparticles of a high purity (e.g., selected crystalline phase) and a narrow size distribution.

### Alpha-Alumina (α-Al₂O₃) Nanoparticles

α-Al₂O₃ nanoparticles are used in the present conjugates. The inventors have found that α-Al₂O₃ nanoparticles can be used to promote cross-presentation of antigens by antigen-presenting cells. For example, conjugation of ovalbumin (OVA) to α-Al₂O₃ nanoparticles was found to have resulted in efficient cross-presentation of OVA antigen *in vitro* (see Example 3). Also, DCs pulsed with α-Al₂O₃ nanoparticle (60 nm)-OVA conjugates were found to cross-present OVA antigens to naive T cells more efficiently both *in vitro* and *in vivo* compared to DCs loaded with soluble OVA (see Example 4). Without wishing to be bound to any particular theory, it is believed that the enhanced cellular immune response is achieved by the ability of α-Al₂O₃ nanoparticles (∼ 10-100 nm) to diverge the antigens, upon conjugation, from the endosome-to-lysosome protein degradation pathway (the usual degradation pathway for peptide antigens) to the endosome-to-autophagosome protein degradation pathway.

It is disclosed that in addition to autophagosomes, other antigens (for example, various protein or peptide antigens) can be conjugated to α-Al₂O₃ nanoparticles having a diameter ranging from 5 nm to 150 nm (e.g., ranging from about 10 nm to about 100 nm), where the resulting conjugates can induce an increased or enhanced T cell-mediated immune response via more efficient cross-presentation of such antigens by antigen-presenting cells. For example, in place of the autophagosomes described herein (i.e., DRibble containing DRiPs or SLiPs), α-Al₂O₃ nanoparticles having a diameter ranging from about 5 nm to about 150 nm can be used to conjugate with a DRiP, a SLiP, or an immunogenic fragment thereof. It is disclosed that α-Al₂O₃ nanoparticles having a diameter ranging from about 5 nm to about 150 nm also can be used to conjugate with whole tumor cells, where the whole tumor cells can be obtained as described hereinabove.

Furthermore, while DCs are used in various examples herein to exemplify antigen-presenting cells (APC), other examples of APCs include monocytes, macrophages, B cells, and Langerhans cells. An antigen-presenting cell carries on its surface antigen bound to MHC class I or class II molecules and presents the antigen in this context to T cells.

Accordingly, the present teachings can be extended more generally to nanoparticle-antigen conjugates, where the nanoparticles and the conjugation chemistry between the nanoparticle and the antigen are as described herein in accordance with nanoparticle-autophagosome conjugates. The nanoparticle-antigen conjugates may include a tumor-specific antigen or a pathogen-specific antigen. In certain embodiments, the nanoparticle-antigen conjugates according to the claims can include a whole tumor cell, where the whole tumor cell functions as the antigen. The nanoparticle-antigen conjugates can include substances derived from tumor cells such as a DRiP, a SLiP, or an immunogenic fragment thereof. The nanoparticle-antigen conjugates can include a tumor-specific or pathogen-specific DRiP (or an immunogenic fragment thereof). The nanoparticle-antigen conjugates described herein generally are able to induce a more significant T cell response (such as a CD4⁺ response or a CD8⁺ response) than a B cell response (which results in the production of specific antibodies to the antigen).

### Nanoparticle-Autophagosome Conjugates

The present nanoparticle-autophagosome conjugates can be prepared by various coupling reactions known in the art. For example, aldehyde-amine, phosphine-amide, maleimide-thiol, aldehyde-hydrazine, azide alkyne Huisgen cycloaddition (click chemistry), coupling, or intein-mediated protein ligation can be used.

In various embodiments, the surface of the alumina nanoparticles can be modified or functionalized with reactive groups such as NH₂, COO⁻, OH, and SH, then optionally further modified with a linker having a terminal functional group that can react with a membrane protein or a membrane lipid of the autophagosome. In some embodiments, the autophagosome also can be modified with a linker. In certain embodiments, the alumina nanoparticle and the autophagosome are modified with different biheterofunctional linkers, each of which includes a terminal functional group that allows the alumina nanoparticle and the autophagosome to react specifically to each other. In certain embodiments, an autophagosome can be attached to the surface of a nanoparticle via one or more linkers that do not include thioether bonds.

Using a multi-step coupling reaction between the nanoparticle and the autophagosome can bring forth several advantages despite the additional steps involved. For example, by functionalizing the autophagosomes with a linker, unfunctionalized autophagosomes can be removed in an optional purification step, hence increasing the yield of the reaction with the activated nanoparticles. By varying the molar amount of activating agents and/or linkers used in the surface modification step(s) of the nanoparticles, the surface packing density of the nanoparticles and in turn the size of the nanoparticle-autophagosome conjugates can be controlled.

In particular embodiments, an autophagosome can be attached to an alumina nanoparticle according to the claims via a hydrazone bond (-NH-N=CR-). The surface of an alumina nanoparticle can be activated with one or more benzaldehyde groups, while autophagosomes can be functionalized with hydrazine functional groups. Upon reaction between the hydrazine and the aldehyde groups which occurs at neutral pH, one or more autophagosomes are attached to the surface of an alumina nanoparticle via hydrazone bonds.

More specifically, the surface of the alumina nanoparticles can be first activated with amine groups by reacting the nanoparticles with an amine such as 4-aminophenol in deionized water. The activated nanoparticles then can be modified with an aldehyde-containing linker such as succinimidyl 4-formylbenzoate (SFB). Autophagosomes can be modified with a hydrazine-containing linker such as succinimidyl 4-hydrazinonicotinate acetone hydrazone (SANH).

The resulting hydrazone bonds attaching an autophagosome to an alumina nanoparticle are stable at neutral pH but are acid-sensitive. Accordingly, the nanoparticle-autophagosome conjugates have good shelf-life at neutral pH, i.e., can be stored for an extended period of time (3 months or more) at neutral pH prior to use; but following administration into a subject, the conjugates can be readily lysed by lysosomes, and release active, immunogenic autophagosomes. How easily antigens released from particles could determine the efficiency of antigen presentation.

### Immunogenic compositions

Immunogenic compositions and methods of producing such compositions are provided by the present teachings. Immunogenic compositions are those that can stimulate or elicit an immune response by a subject's immune system, such as stimulating the production of a T-cell response in the subject, for example a T-cell response against a tumor-associated antigen or a pathogen-associated antigen (such as a viral-associated antigen). Exemplary immunogenic compositions include vaccines, which can be used prophylactically or therapeutically. In various examples, immunogenic compositions according to the present teachings can include one or more nanoparticle-antigen conjugates described herein, antigen-presenting cells loaded with one or more nanoparticle-antigen conjugates (conjugate-loaded APCs) described herein, and combinations thereof.

In addition to the nanoparticle-antigen conjugates and/or conjugate loaded APCs according to the claims the present immunogenic compositions can include other agents, such as one or more pharmaceutically acceptable carriers, immunostimulants, anti-neoplastic chemotherapeutic agents, or combinations thereof. One particular example of an immunostimulant is anti-OX-40 antibody. Another example is an ssRNA, such as an ssRNA single strand oligoribonucleotides. A further example is a cytokine, such as GM-CSF.

It is disclosed that an immunogenic composition may be generated by producing a population of nanoparticle-autophagosome conjugates using the methods described herein, and then preparing a composition that includes the nanoparticle-autophagosome conjugates. The immunogenic composition can include a population of nanoparticle-autophagosome conjugates suspended in a pharmaceutically acceptable fluid carrier.

It is disclosed that methods of generating an immunogenic composition may include contacting (incubating) a population of nanoparticle-autophagosome conjugates with an antigen-presenting cell (APC), thereby generating an immunogenic composition that includes conjugate-loaded APCs. In particular examples, the conjugate-loaded APCs are isolated from the conjugates, for example by washing the conjugate-loaded APCs, and the isolated conjugate-loaded APCs form an immunogenic composition. The antigen-presenting cells may be dendritic cells (DC), and the immunogenic composition may include conjugate-loaded DCs. Methods of obtaining or generating APCs from a subject are known in the art. For example, APCs can be obtained from a blood sample from a mammal. More specifically, monocytes obtained from blood sample can be cultured to generate DCs. In particular examples, APCs (or precursors thereof) are obtained from the subject in whom an immune response is to be stimulated prior to administering an immunogenic composition. For example, the method can include isolating peripheral blood mononuclear cells (PBMCs) from the subject, wherein the PBMCs are used to obtain or generate APCs. In particular disclosed herein, examples, nanoparticle-autophagosome conjugates according to the present teachings are incubated with APCs to generate conjugated-loaded APCs. A population of such conjugate-loaded DCs are then suspended in a pharmaceutically acceptable fluid carrier to provide an immunogenic composition.

Various pharmaceutically acceptable fluid carriers are known in the art. In general, the nature of the fluid carrier will depend on the particular mode of administration being employed. For instance, parenteral formulations can include injectable fluids that include pharmaceutically and physiologically acceptable fluids such as water, physiological saline, balanced salt solutions, aqueous dextrose, glycerol or the like as a vehicle. In addition to biologically-neutral carriers, pharmaceutical compositions to be administered can contain minor amounts of non-toxic auxiliary substances, such as wetting or emulsifying agents, preservatives, and pH buffering agents and the like, for example, sodium acetate or sorbitan monolaurate, sodium lactate, potassium chloride, calcium chloride, and triethanolamine oleate. For example, the immunogenic compositions described herein can include a pharmaceutically acceptable fluid carrier selected from saline, an aqueous electrolyte solution, and a buffered aqueous solution.

### Administration

As used herein, the term "administration" refers to the act of providing or giving a subject an agent, such as an immunogenic composition disclosed herein, by any effective route. Exemplary routes of administration include, but are not limited to, oral, injection (such as subcutaneous, intramuscular, intradermal, intraperitoneal, and intravenous), sublingual, rectal, transdermal, intranasal, vaginal and inhalation routes. As used herein, a "subject" can be any living multi-cellular vertebrate organisms, particularly, mammals. In various embodiment, a subject can be a human or a non-human mammal (such as a laboratory or veterinary subject).

Any mode of administration can be used for administering a therapeutic agent, such as the nanoparticle-antigen conjugates (e.g., the α-Al₂O₃ nanoparticle-autophagosome conjugates) and the immunogenic compositions disclosed herein. Those skilled in the art, such as a treating physician, can determine an appropriate route of administration. In some embodiments, administration of an immunogenic composition is subcutaneous or intradermal. In another example, administration of a lymphodepletion agent is intravenous.

The conjugates and the immunogenic compositions can be administered to a subject in therapeutically effective amounts. As used herein, a "therapeutically effective amount" refers to an amount of an agent that alone, or together with a pharmaceutically acceptable carrier or one or more additional therapeutic agents, induces the desired response. A therapeutic agent, such as an immunogenic composition that includes the present nanoparticle-autophagosome conjugates, can be administered in therapeutically effective amounts that stimulate a protective immune response, for example against a target antigen. The minimum therapeutically effective amount of a therapeutic agent can be determined in many different ways, such as by assaying for an increase in an immune response, for example by assaying for improvement of a physiological condition of a subject having a disease (such as a tumor or pathogen infection). Therapeutically effective amounts also can be determined through various *in vitro, in vivo* or *in situ* assays.

Therapeutic agents can be administered in a single dose, or in several doses, for example weekly, monthly, or bimonthly, during a course of treatment. However, the therapeutically effective amount of a therapeutic agent can be dependent on the source applied, the subject being treated, the severity and type of the condition being treated, and the manner of administration.

In one example, it is an amount sufficient to partially or completely alleviate symptoms of an infectious disease within a subject, or to decrease infection by a pathogen. Treatment can involve only slowing the progression of the disease temporarily, but can also include halting or reversing the progression of the disease permanently, as well as preventing disease in the first place. For example, a pharmaceutical preparation can decrease one or more symptoms of infectious disease, for example decrease a symptom by at least 20%, at least 50%, at least 70%, at least 90%, at least 98%, or even at least 100%, as compared to an amount in the absence of the pharmaceutical preparation.

In another example, it is an amount sufficient to partially or completely alleviate symptoms of a tumor in a subject. Treatment can involve only slowing the progression of the tumor temporarily, but can also include halting or reversing the progression of the tumor permanently. For example, a pharmaceutical preparation can decrease one or more symptoms of the tumor (such as the size of the tumor or the number of tumors), for example decrease a symptom by at least 20%, at least 50%, at least 70%, at least 90%, at least 98%, or even at least 100%, as compared to an amount in the absence of the pharmaceutical preparation.

Accordingly, in certain embodiments, a therapeutically effective amount of the present immunogenic composition is administered in a single unit dose. As used herein, a "unit dose" refers to a physically discrete unit containing a predetermined quantity of an active agent calculated to produce individually or collectively a desired effect such as an immunogenic effect. A single unit dose or a plurality of unit doses can be used to provide the desired effect, such as an immunogenic effect. In certain embodiments, a therapeutically effective amount of the present immunogenic composition is administered in at least two unit doses, such as at least three unit doses, four unit doses, or five unit doses, over a period of at least 60 days, at least 90 days, at least 180 days, or at least 365 days.

### Stimulating an Immune Response Against a Tumor

It is disclosed that the present conjugates, conjugate-loaded APCs, and/or an immunogenic composition including the present conjugates and/or conjugate-loaded APCs may be used to stimulate an immune response against a tumor, such as tumor-derived DRiPs. Non-limiting tumors include benign tumors such as pituitary adenomas and gastrointestinal adenomatous polyps. Exemplary malignant tumors, include, but are not limited to, breast cancer, lung cancer, renal cell carcinoma, or liver cancer. Tumors can be solid or hematological. Examples of hematological tumors include, but are not limited to, leukemias, including acute leukemias (such as acute lymphocytic leukemia, acute myelocytic leukemia, acute myelogenous leukemia and myeloblastic, promyelocytic, myelomonocytic, monocytic and erythroleukemia), chronic leukemias (such as chronic myelogenous leukemia, and chronic lymphocytic leukemia), myelodysplastic syndrome, and myelodysplasia, polycythemia vera, lymphoma, (such as Hodgkin's disease, all forms of non-Hodgkin's lymphoma), multiple myeloma, Waldenstrom's macroglobulinemia, and heavy chain disease. Examples of solid tumors, such as sarcomas and carcinomas, include, but are not limited to, fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, pancreatic cancer, breast cancer, lung cancer, ovarian cancer, prostate cancer, hepatocellular carcinoma, squamous cell carcinoma, basal cell carcinoma, adenocarcinoma, sweat gland carcinoma, sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, bladder carcinoma, melanoma, and CNS tumors (such as a glioma, astrocytoma, medulloblastoma, craniopharyogioma, ependymoma, pinealoma, hemangioblastoma, acoustic neuroma, oligodendroglioma, menangioma, meningioma, neuroblastoma and retinoblastoma).

Although tumor cells produce DRiPs, the DRiPs are not efficiently cross-presented due to their rapid degradation by proteasomes. Because most tumor cells express MHC class I but not class II molecules on their surface, and because cross-presentation favors long-lived proteins and misses DRiPs and SLiPs, a much larger array of antigen repertoire presented by tumor cells are not cross-presented by APCs. In contrast, DRibbles produced by tumor cells due to contact with a proteasome inhibitor and lysosomal blockade (for example in combination with an autophagy inducer) are loaded by APCs, thereby permitting cross-presentation of tumor-derived DRiPs by the APCs.

Therefore, in some embodiments, the present teachings provide the conjugates and compositions according to the claims for use in methods of stimulating an immune response against a tumor in a subject by administering to the subject a therapeutically effective amount of a conjugate and/or a conjugate-loaded APC disclosed herein (either alone or in combination with other agents, such as an immunostimulant like anti-OX40 antibody), thereby stimulating an immune response against one or more tumor-derived DRiPs. In certain embodiments, the method further includes administration of therapeutically effective amounts of an agent that reduces or inhibits tumor or stromal cell inhibitory molecules, such as PD-1.

In particular embodiments, autophagosome used to prepare the present nanoparticle-autophagosome conjugates are generated from a tumor cell of the same type as is present in the subject, for example using the methods described herein. For example, if the subject has lung cancer, the autophagosomes are produced from a lung cancer cell. In some examples, the tumor cells used to produce the autophagosomes are obtained from the subject to be treated. Therefore, in some examples, the method can include obtaining a sample that includes tumor cells from the subject prior to administration of an immunogenic composition to the subject. The method can further include culturing the tumor cells under conditions sufficient to permit viability, growth, or expansion of the tumor cells. However, as noted above, not all primary tumor cells grow well in culture. As a result, in some examples the tumor cells used to produce the autophagosomes can be obtained from a tumor cell line of the same cell type as the tumor in the subject.

In certain embodiments, the method is a method of stimulating an immune response against a tumor cell in a subject. In some examples, the method includes exposing tumor cells to a proteasome inhibitor ex vivo under conditions sufficient to produce autophagosomes (DRibbles) by the tumor cells, wherein the tumor cells are the same type of tumor cells present in the subject. In some examples, the tumor cells are also incubated with an autophagy inducer. In some examples, the tumor cells are obtained from the same subject to be treated. The autophagosomes are isolated from the treated tumor cells, modified with a linker, and reacted with an alumina nanoparticle having one or more linkers on its surface that is reactive to the linkers on the autophagosomes, to provide a nanoparticle-autophagosome conjugate. The nanoparticle-autophagosome conjugate is then administered to the subject at a therapeutic dose, for example alone or in the presence of an adjuvant or other immunostimulatory agent, or an anti-tumor agent, thereby stimulating an immune response against one or more DRiPs. In some examples, the conjugates are incubated with an APC obtained from peripheral blood mononuclear cells (PBMCs) from the subject under conditions sufficient for the APC to present one or more DRiPs, thereby generating conjugate-loaded APCs. The resulting conjugate-loaded APCs are administered to the subject at a therapeutic dose (alone or in the presence of another therapeutic agent, such as an immunostimulatory agent or an anti-tumor agent), thereby stimulating an immune response against one or more DRiPs.

The disclosed methods can be used to treat a subject having one or more tumors. For example, administration of the present nanoparticle-autophagosome conjugates, conjugate-loaded APCs, or an immunogenic composition including the present nanoparticle-autophagosome conjugates and/or conjugate-loaded APCs, can reduce one or more symptoms of a tumor, such as the size of a tumor, the number of tumors, or prevent metastasis of a tumor.

### Lymphodepletion and Reconstitution

In addition to the initial activation of tumor-reactive T cells, a long-term persistence of these activated T cells *in vivo* can be obtained. For example, to increase the initial expansion and late persistence of tumor-reactive cytolytic T- lymphocytes and helper T-lymphocytes, prior to administration of a therapeutically effective amount of the present conjugates, conjugate-loaded APCs, or combinations thereof (such as an immunogenic composition containing both the conjugates and the conjugate-loaded APCs), subjects can be administered one or more agents that alone, or in combination, substantially lymphodeplete the subject. The lymphodepletion agents can be administered under conditions sufficient to achieve lymphodepletion in the subject. In particular examples, a subject is substantially lymphodepleted if the number of lymphocytes in the subject decreases by at least 50%, such as at least 90%, following administration of the lymphodepletion agent.

In particular examples, significantly reducing the white blood cell count in a subject having a tumor prior to vaccination with the present conjugates, or conjugate-loaded APCs, can elicit a stronger immune response and more tumor cells can be destroyed than if no lymphodepletion agent were administered.

In one example, a lymphodepletion agent is an anti-neoplastic chemotherapeutic agent. Examples of lymphodepletion agents include, but are not limited to fludarabine, cyclophosphamide, or combinations thereof. The specific lymphodepletion agent(s) and their appropriate dosages can be selected by a treating physician depending on the subject to be treated.

In particular examples, the method further includes lymphodepleting subjects, followed by reconstituting the immune system of the subject. For example, prior to lymphodepletion and administration of an immunogenic composition according to the present teachings, blood cells (such as monocytes and macrophages) can be obtained from the subject, for example by using leukapheresis. The isolated blood cells can be frozen until a time appropriate for introducing the blood cells into the subject. For example, thawed lymphocytes can be administered to the subject at the same time as the immunogenic composition is administered, or shortly before or after administration of the immunogenic composition. In particular embodiments, such reconstitution of the immune system can enhance stimulation of the immune system.

### Stimulating an Immune Response Against a Pathogen

The present conjugates, conjugate-loaded APCs, or an immunogenic composition including the present conjugates and/or conjugate-loaded APCs disclosed herein can be used to stimulate an immune response against a pathogen, such as pathogen-derived DRiPs. Examples of pathogens include, but are not limited to, viruses, bacteria, protozoa, and fungi, such as HIV, influenza, and Listeria.

Therefore, in some embodiments, the present teachings provide the conjugates and compositions according to the claims for use in methods of stimulating an immune response against a tumor in a subject by administering to the subject a therapeutically effective amount of a nanoparticle-autophagosome conjugate disclosed herein, thereby stimulating an immune response against one or more pathogen-derived DRiPs. The autophagosomes used to prepare the nanoparticle-autophagosome conjugates are generated from cells infected with the target pathogen. For example, the autophagosomes can be produced from a cell infected with one or more desired pathogens (or transduced with a vector encoding one or more pathogen-specific antigens) using the methods described herein.

The immunogenic compositions disclosed herein can be used to treat a subject, for example by preventing infection of the subject by a pathogen, or by treating an existing infection in the subject, such as an infectious disease. In some embodiments, treatment of the subject is prophylactic, for example, to prevent future infection or future infectious disease in the subject. In some embodiments, treatment includes reducing one or more symptoms associated with an infectious disease, such as reduction of vomiting, diarrhea, fever or chills, or increasing the number of functional lymphocytes. The autophagosomes of the conjugates or the conjugate-loaded APCs used will correspond to the infectious disease to be treated.

Particular examples of infectious diseases caused by a bacterium include, but are not limited to, tuberculosis (caused by Mycobacterium tuberculosis); heartworm (caused by Dirofilaria immitis); gastric disorders (caused by Helicobacter pylori); intestinal disorders (such as those caused by Escherichia coli); pulmonary disorders (such as those caused by Haemophilus influenzae) and pneumoniae (such as those caused by Streptococcus pneumoniae, Staphylococcus aureus, Pseudomonas aeruginosa, and Klebsiella pneumoniae).

Particular examples of infectious diseases caused by a virus include, but are not limited to, cytomegalovirus (CMV) pneumonia, enteritis and retinitis; Epstein-Barr virus (EBV) lymphoproliferative, disease; chicken pox/shingles (caused by Varicella zoster virus, VZV); HSV-1 and -2 mucositis; HSV-6 encephalitis, BK-virus hemorrhagic cystitis; viral influenza; pneumonia from respiratory syncytial virus (RSV); AIDS (caused by HIV); cervical cancer (caused by human papillomavirus); and hepatitis A, B or C.

Particular examples of infectious diseases caused by a protozoa include, but are not limited to, malaria (caused by Plasmodium falciparum); trypanosoma and Chagas' disease (caused by Trypanosoma cruzi), toxoplasma; leishmaniasisa and kalaazar (caused by Leishmania); giardiasis (caused by Giardia); Cryptosporidium; balantidiasis (caused by Balantidium coli); strongyloidiasis (caused by Strongyloides stercoralis); roundworms such as Trichuris, hookworm, and Strongyloides; and capillariasis (caused by Capillariasis).

Particular examples of infectious diseases caused by a fungus include, but are not limited to, thrush (caused by Candida albicans); cryptococcemia (caused by Cryptococcus); histoplasmosis (caused by Histoplasma), and aspergillosis (caused by Aspergillus spp.).

The type of immune response (whether against a tumor or a pathogen) stimulated by the present conjugates and/or conjugate-loaded APCs can be mainly immune cell-mediated (as opposed to antibody-mediated). The extent of the immune response (or the immunogenicity of the immunogenic compositions described herein) can be measured, for example, by the ability to bind to an appropriate MHC molecule (such as an MHC Class I or II molecule) and to induce a T-cell response or to induce a B-cell or antibody response, for example, a measurable cytotoxic T-cell response or a serum antibody response to a given epitope. Immunogenicity assays are well-known in the art and are described, for example, in Paul, Fundamental Immunology, 3rd ed., 243-247 (Raven Press, 1993) and references cited therein.

### Examples

Aspects of the present teachings can be further understood in light of the following examples. In addition, all *in vitro* cross-presentation experiments below are representative of at least three independently performed experiments. The *in vivo* cross-presentation (three mice per group) and tumor therapy experiments (five mice per group) below are representative of two independent experiments. Examples which are not encompassed under the scope of the claims do not form part of the invention and are cited as reference examples.

### Example 1 Preparation and characterization of alumina nanoparticle bioconjugates

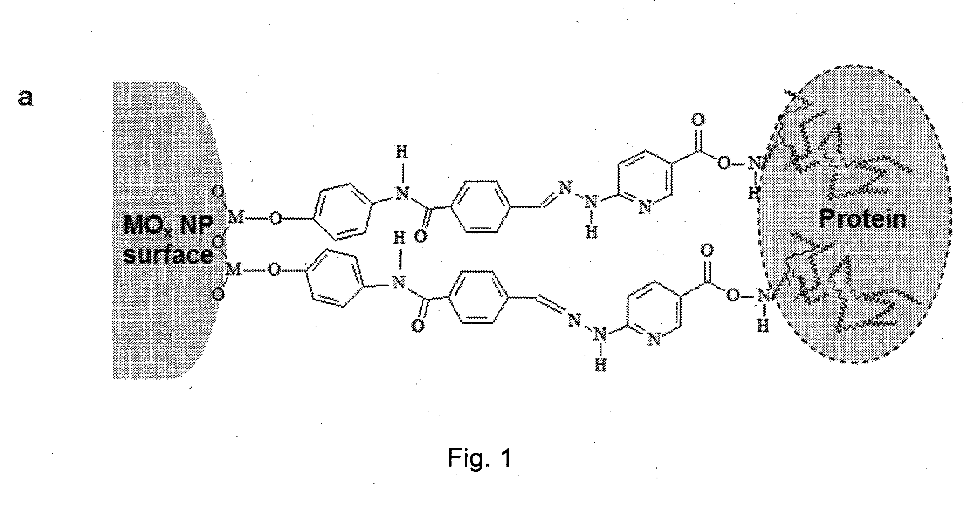

Ovalbumin was used as an exemplary protein antigen to illustrate the bioconjugates of the present teachings. Specifically, soluble ovalbumin (OVA) was conjugated to various metal oxide nanoparticles (MOₓ NPs) via chemoselective ligation between hydrazine-modified NPs and OVA modified with aromatic aldehyde. Fig. 1a shows the structure of an exemplary protein antigen conjugated to a metal oxide nanoparticle (MOₓ NP) according to the present teachings.

Metal oxide nanoparticles including α-Al₂O₃ NPs (60 nm and 200 nm, Nanoamor) (2 mg/mL), anatase TiO₂ NPs (25 nm and 100 nm, Sigma-Aldrich) (2 mg/mL), and α-Fe₂O₃ NPs (25 nm, Nanoamor) (2 mg/mL) were functionalized with amine groups (-NH₂) by reacting with 4-aminophenol (Sigma) (50 µg/mL) in deionized water at 90 °C for two hours. The amine functionalized MOₓ NPs were washed by centrifugation at 9000 × g for one hour and re-suspended in deionized water. Subsequently, the amine-functionalized MOₓ NPs were further modified using succinimidyl 4-formylbenzoate (SFB). Soluble ovalbumin (Sigma) (2 mg/mL) was modified using succinimidyl 4-hydrazinonicotinate acetone hydrazone (SANH). After mixing equal volumes of a suspension of SFB-modified MOₓ NPs (10 mg/mL) and a solution of SANH-modified OVA (2 mg/mL), the metal oxide (MOₓ) NP-OVA conjugates were purified by centrifugation at 9000 × g for one hour and re-suspended in phosphate buffered saline (PBS). The amount of OVA proteins conjugated to MOₓ NPs and the amount of OVA proteins remaining in the supernatant were determined using the bicinchoninic acid (BCA) assay. The amounts of OVA conjugated to 1 mg/mL α-Al₂O₃ NPs (60 nm), α-Al₂O₃ NPs (200 nm), anatase TiO₂ NPs (25 nm), anatase TiO₂ NPs (100 nm), and α-Fe₂O₃ NPs (25 nm) were determined to be about 0.089 mg/mL, 0.086 mg/mL, 0.263 mg/mL, 0.030 mg/mL, and 0.245 mg/mL, respectively. The endotoxin content of the various MOₓ NPs was determined using a Limulus Amebocyte Lysate Kit (QCL-1000, BioWhittaker). The endotoxin content was below the level that is required to activate dendritic cells (less than 0.05 EU/µg NPs).

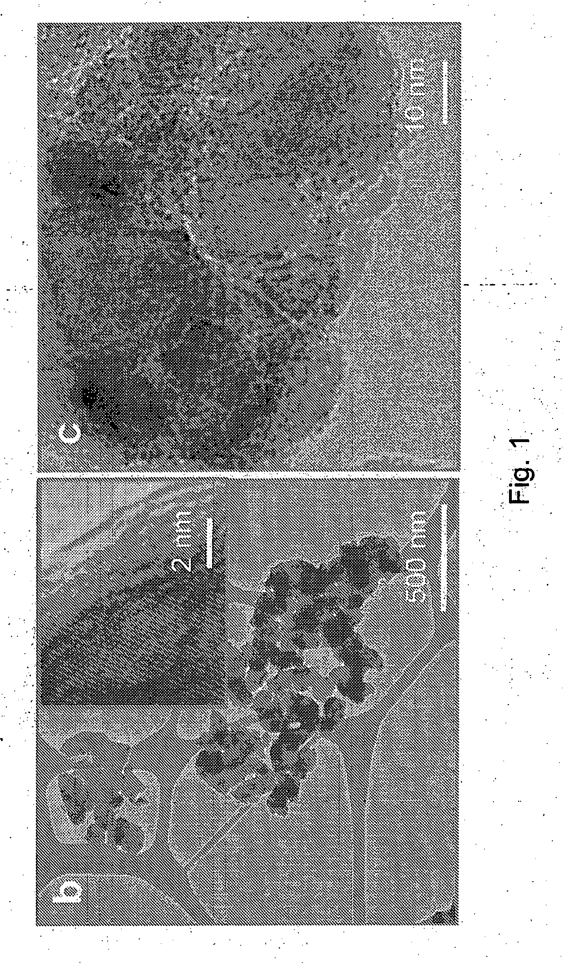

The shape, internal structure and chemical composition of MOₓ NPs and MOₓ NP-OVA conjugates were analyzed by transmission electron microscopy (TEM) analysis using an FEI Tecnai F20 TEM equipped with an energy dispersive X-ray (EDX) spectrometer. The TEM analysis shows that the single crystalline α-Al₂O₃ NPs with a clean surface (Fig. 1b) were coated with an amorphous layer after conjugation (Fig. 1c).

### Example 2: Uptake of α-Al₂O₃ NP-OVA conjugates by dendritic cells

The uptake of α-Al₂O₃ NP-OVA conjugates by bone marrow-derived immature dendritic cells (DCs) was monitored using a Zeiss Axiovert 40 CFL inverted microscope with an excitation source at 488 nm. α-Al₂O₃ NP-OVA conjugates were labeled using Alexa 488 (Invitrogen). Bright field and fluorescent images of live cells were acquired and then overlaid using Photoshop imaging software.

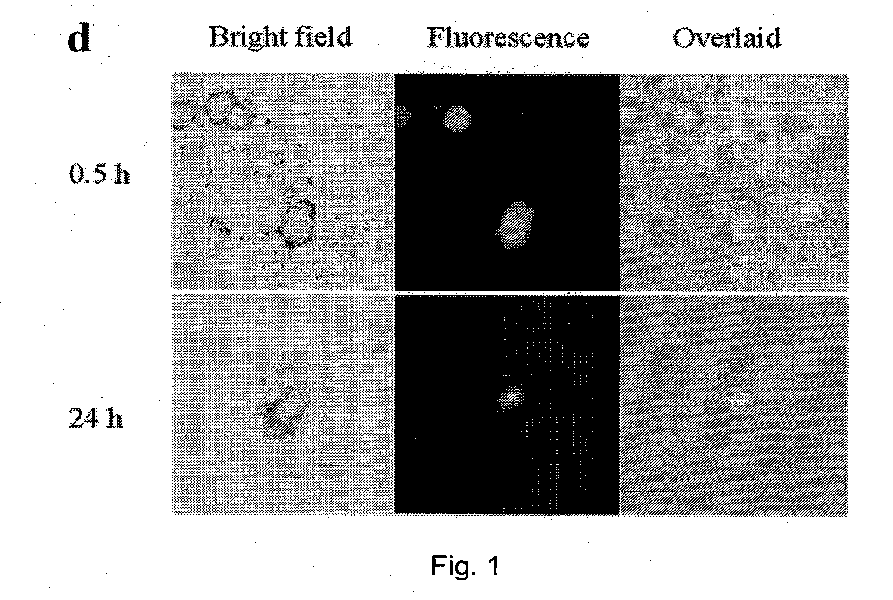

The fluorescence microscopy images obtained show that α-Al₂O₃ NP-OVA conjugates could be phagocytized by DCs efficiently (Fig. 1d). Initially, the internalized α-Al₂O₃ NP-OVA conjugates were near the plasma membrane. Ultimately, they migrated to the perinuclear region of the DCs over an incubation time of 24 hours. This is consistent with reported observation that the translocation of payload carrying vesicles is along microtubules and towards the microtubule-organizing centers of DCs.

### Example 3: Expression of major histocompatibility complex class I (MHC I) molecules

The efficiency of cross-presentation of OVA by α-Al₂O₃ NPs was evaluated with the surface expression of MHC I peptide complexes (K^{b}-SIINFEKL) on dendritic cells (DCs).

Immature DCs were derived from bone marrow of C57BL6 mice (purchased from Charles River Laboratories, Wilmington, MA) after in vitro culture with complete media supplemented with 20 µg recombinant GM-CSF (Peprotech). DCs were incubated with OVA and α-Al₂O₃ NP-OVA conjugates for 18 hours. The washed cells were stained using biotin-conjugated 25D-1.16 mAb, which recognizes the K^{b}-SIINFEKL complexes (OVA₂₅₇₋₂₆₄) derived from OVA proteins. The K^{b}-SIINFEKL complexes were quantified by a BD FACS Aria^{™} II flow cytometer after staining with PE-conjugated strepavidin.

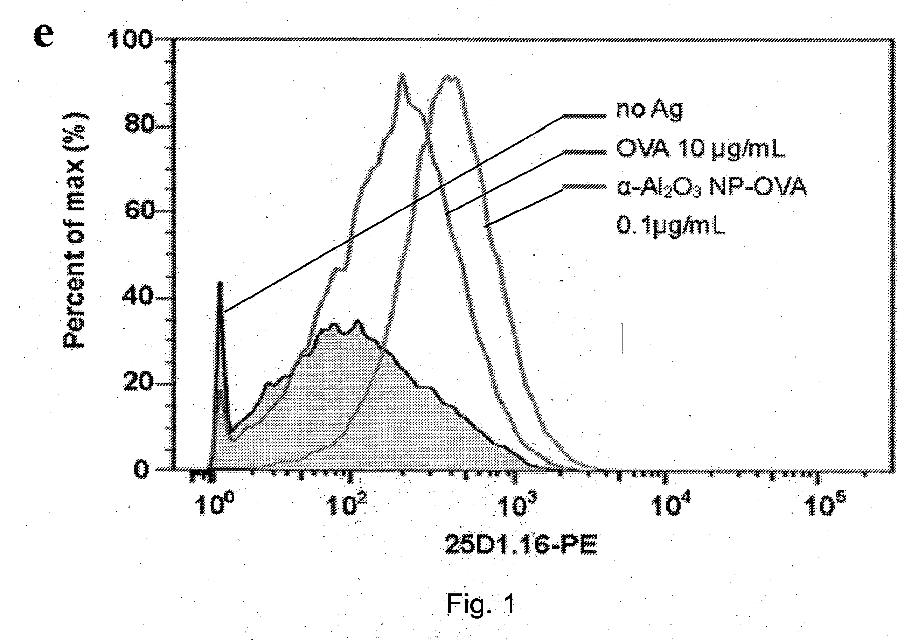

As shown in Fig. 1e, DCs loaded with α-Al₂O₃ NP-OVA conjugates yielded a higher level of K^{b}-SIINFEKL complexes than the DCs loaded with soluble OVA, conforming that OVA proteins are more efficiently cross-presented when conjugated to α-Al₂O₃ NPs than in soluble form.

### Example 4: In vitro and in vivo activation of CD8⁺ T cells

DCs loaded with OVA antigens alone and those loaded with OVA antigens conjugated to α-Al₂O₃ NPs (200 nm or 60 nm), anatase TiO₂ NPs (100 nm or 25 nm), and α-Fe₂O₃ NPs (25 nm), respectively, were obtained and their respective abilities to stimulate naive OVA-specific CD8⁺ T cells *in vitro* and *in vivo* were observed. Further comparisons were tested with DCs loaded with a mixture of α-Al₂O₃ NPs and OVA, which was prepared by mixing equal volumes of OVA (0.2 mg/mL) and α-Al₂O₃ NPs (2 mg/mL) suspensions for 12 hours.

*In vitro* cross-presentation of OVA was measured by a dye dilution assay of CFSE-labeled naive OT-I T cells. OVA, α-Al₂O₃ NP-OVA conjugates, anatase TiO₂ NP-OVA conjugates, α-Fe₂O₃ NP-OVA conjugates, or mixtures of α-Al₂O₃ NPs and OVA (0.1 µg/mL OVA loaded for each sample) were incubated with 5 × 10⁵ bone marrow derived DCs or DC2.4 cells for 6 hours, washed three times, and co-incubated for 60 hours with 1 × 10⁶ CFSE-labeled OT-I T cells. The percentage of divided Thy1.1⁺ OT-I CD8⁺ T cells was measured by flow cytometry analysis.

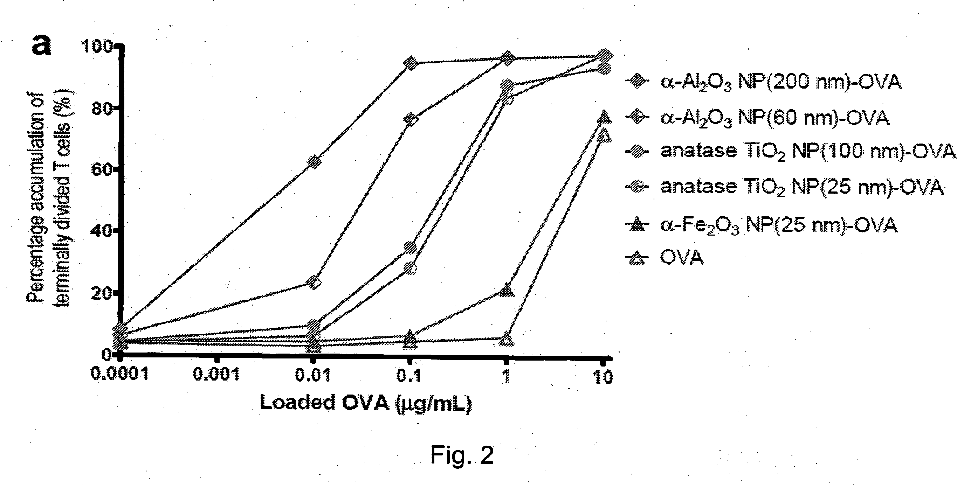

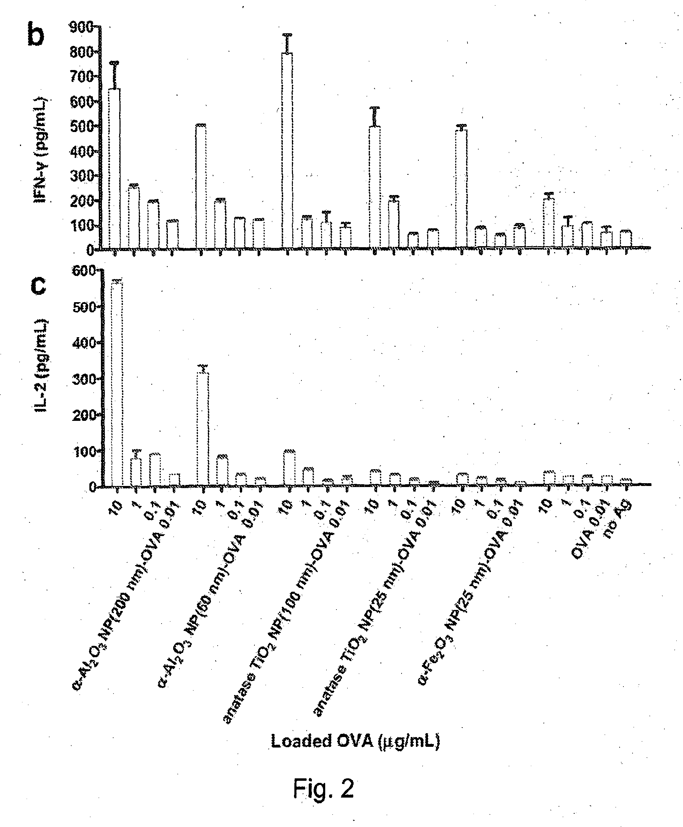

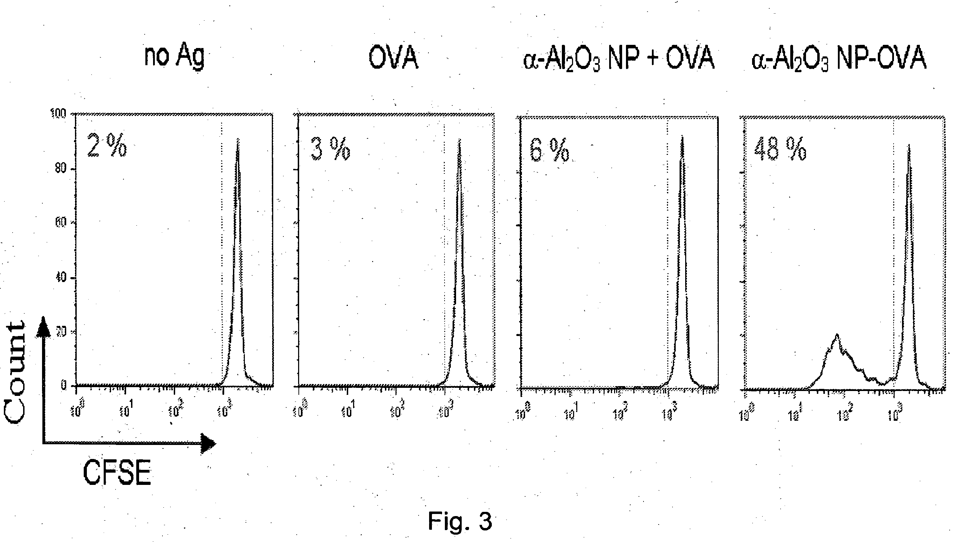

In the *in vitro* study, it was observed that DCs loaded with α-Al₂O₃ NP (60 nm or 200 nm)-OVA conjugates induced a dose-dependent proliferation of Thy1.1⁺ OT-1 CD8⁺ T cells (Fig. 2a). In addition, it was observed that DCs loaded with a mixture of OVA and α-Al₂O₃ NPs were inefficient to activate naive T cells (Fig. 3). This suggests that delivery of OVA and α-Al₂O₃ NPs into the same intracellular compartment of DC is the critical procedure for efficient cross-presentation. The half-maximal effective concentration (*EC*₅₀) for α-Al₂O₃ NP (60 nm)-OVA conjugates and α-Al₂O₃ NP (200 nm)-OVA conjugates were approximately 10 ng/mL and 1.3 ng/mL, respectively, which are approximately 500-1000 folds more efficient than OVA (5 µg/mL). Further, DCs cross-presented OVA more effectively when the OVA is conjugated to α-Al₂O₃ NPs than to TiO₂ NPs or α-Fe₂O₃ NPs regardless of their sizes, indicating that the chemical properties of α-Al₂O₃ NPs also are involved in enabling efficient cross-presentation. In addition, only α-Al₂O₃ NP-OVA conjugates were able to stimulate significant production of both IFN-γ and IL-2 (Figs. 2b and 2c).

Moreover, it was found that DCs loaded with α-Al₂O₃ NP-OVA conjugates are superior to DCs loaded with OVA immunocomplexes (OVA-IC) or OVA in the presence of a TLR agonist (OVA+MPL) at stimulating naive OT-I T cells *in vitro.* DC2.4 cells (10⁵) were loaded with various amounts (0.001, 0.01, 0.1, 1,10 µg) of OVA, α-Al₂O₃ NP-OVA conjugates, OVA-IC, and OVA in the presence of 100 ng/mL MPL (a TRL agonist from Avanti Polar Lipids, Inc.) and used to stimulate CFSE-labeled naïve OT-I T cells. For the preparation of immunocomplexes of OVA/anti-OVA, equal amount of OVA (2 mg/mL) and goat-anti-OVA (2 mg/m) were incubated at room temperate overnight before they were used to load DCs. The percentage of divided Thy1.1⁺ OT-I CD8⁺ T cells was measured by flow cytometry analysis. The experiment was repeated twice, and both iterations showed that the efficiency of cross-presentation mediated by α-Al₂O₃ NP (60 nm)-OVA conjugates was significantly higher than Fc receptor-mediated antigen delivery with OVA/anti-OVA immunocomplexes (IC) or the TLR4 agonist (MPL). The estimated enhancement of cross-presentation was 10 and 100 folds for IC and TLR agonist respectively, which is close to that of OVA/anti-DEC-205 immune conjugates and TLR2 agonist.

For *in vivo* cross-presentation, Thy1.1⁺ OT-I transgenic T cells (10⁵) were transferred to C57BL/6 mice, and OVA, α-Al₂O₃ NP-OVA conjugates, anatase TiO₂ NP-OVA conjugates, α-Fe₂O₃ NP-OVA conjugates, or a mixture of OVA/Imject ® Alum Adjuvant

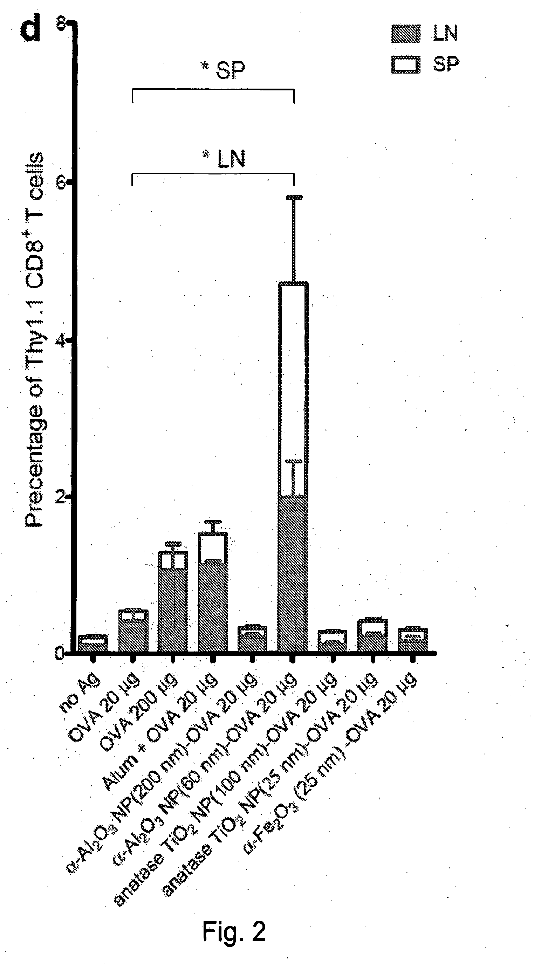

(Thermo Scientific) were injected subcutaneously six hours later. After six days, the lymph nodes and the spleens were collected and processed forming single-cell suspensions. The percentages of cross-primed OT-I CD8⁺ T cells among total cells in the lymph nodes (LN) and in the spleens (SP) was measured using BD FACS Calibur flow cytometry, and the results are plotted in Fig. 2d (* *P* < 0.05).

When mice were injected either with 200 µg of OVA or 20 µg of OVA mixed with the alum adjuvant, the resulting OT-I T-cell expansion was similar. The larger NPs, α-Al₂O₃ NP (200 nm)-OVA conjugates, which induced a strong T cell proliferation in the *in vitro* study, failed to cross-prime naïve T cells *in vivo.* Whereas large quantities of α-Al₂O₃ NP (200 nm)-OVA conjugates were observed to have the tendency of being deposited near the injection sites, the smaller α-Al₂O₃ NP (60 nm)-OVA conjugates disappeared near the site of administration and were able to drain into lymph nodes. The number of Thy1.1⁺ OT-I CD8⁺ T cells in the lymph nodes and spleens of mice injected with α-Al₂O₃ NP (60 nm)-OVA conjugates was substantially higher than that induced by the OVA/Imject® Alum Adjuvant mixture. In addition, consistent with the *in vitro* test, both anatase TiO₂ NP (100 nm or 25 nm)-OVA conjugates and α-Fe₂O₃ NP (25 nm)-OVA conjugates were found to be inefficient in inducing T cell proliferation either in lymph node or in spleen. These results show that α-Al₂O₃ NPs with a diameter of less than 100 nm (e.g., about 60 nm) are more effective antigen carriers and unexpectedly appear to function differently than α-Al₂O₃ NPs with larger diameters or traditional alum adjuvants.

### Example 5: Hypothesized mechanism of cross-presentation of antigens by α-Al₂O₃ NP carriers

Multiple mechanisms have been proposed to explain the adjuvant activity of alum. Interestingly, it has been shown that alum delivers soluble antigens to DCs without being phagocytized. To understand the underlying mechanism by which α-Al₂O₃ NPs induce antigen cross-presentation, confocal microscopy and TEM analysis were used to determine the subcellular localization of internalized α-Al₂O₃ NP-OVA conjugates.

Immunostaining, transfection, and confocal microscopy: DCs (10⁵) were incubated with 2 µl Alexa fluor 488-labeled α-Al₂O₃ NP-OVA conjugates (1 mg/mL α-Al₂O₃ NPs (60 nm) and 0.089 mg/mL OVA) for 6 hours. DCs were fixed using 4 % formaldehyde after wash and permeabilized using 0.2% Triton® X-100 in PBS for 15 minutes. Cells were stained with rabbit anti-LC3 antibody (invitrogen, 0.5 µg/mL) and Alexa fluor 568 labeled donkey anti-rabbit secondary antibody (1 µg/mL). For lysosome tracking, the treated DCs were stained with (1 mM) lyso tracker red DND-99 dye (Invitrogen) without fixing. The stained cells were visualized under an Olympus IX81 inverted microscope fitted with an Olympus Fluoview FV1000 confocal laser microscope system. For DNA transfection, bone marrow-derived immature DCs (10⁵) were incubated with a mixture of 0.1 µg pCMV-tdTomato-LC3 or pCMV-tdTomato-p62 (kindly provided by Dr. T. Johansen, Biochemistry Department, University of Tromsø, Norway) and 0.4 µg polyethylenimine (PEI) vectors (Polysciences) in RPMI media without supplement (50 µl) overnight. After washing three times with PBS, the DCs expressing LC3 or p62 fusion proteins were incubated with Alexa 488-labeled α-Al₂O₃NP-OVA conjugates for 6 hours before imaging. The co-localization of the internalized α-Al₂O₃ NP-OVA with tdtomato-LC3 or td-tomato-p62 fusion proteins was imaged.

TEM analysis: α-Al₂O₃ NP-OVA-pulsed DCs were washed and fixed with 4% paraformaldehyde in 0.25 M PBS buffer (pH 7.4) for one hour at room temperature. The fixed DCs were stained with 0.1 M sodium cacodylate buffer and then 2 % osmium tetroxide in 0.1 M sodium cacodylate buffer. After being dehydrated gradually in ethanol solution, the stained DCs were embedded in epoxy resin at 60 °C overnight. The cross-sections of the embedded DCs were characterized using a Philips/FEI CM120/Biotwin TEM.

When DCs were loaded with α-Al₂O₃ NP-OVA conjugates for 6 hours, confocal images showed that the majority of NPs co-localized with autophagosome marker Atg8/LC320, but not lysosome tracker. Such co-localization was verified with transfection of DCs with a plasmid DNA encoding the LC3-tdTomato fusion protein before loading with NP conjugates as observed from confocal images. Interestingly, α-Al₂O₃ NP conjugates also co-localized with p62 (sequestosome-1), a cargo recognition and targeting molecule for autophagy pathway 21. The important role of p62 for selective autophagy of aggregated proteins, damaged organelles, or intracellular bacterial has been elucidated recently, and the results here suggested p62 played a similar role in autophagy of internalized α-Al₂O₃ NPs.

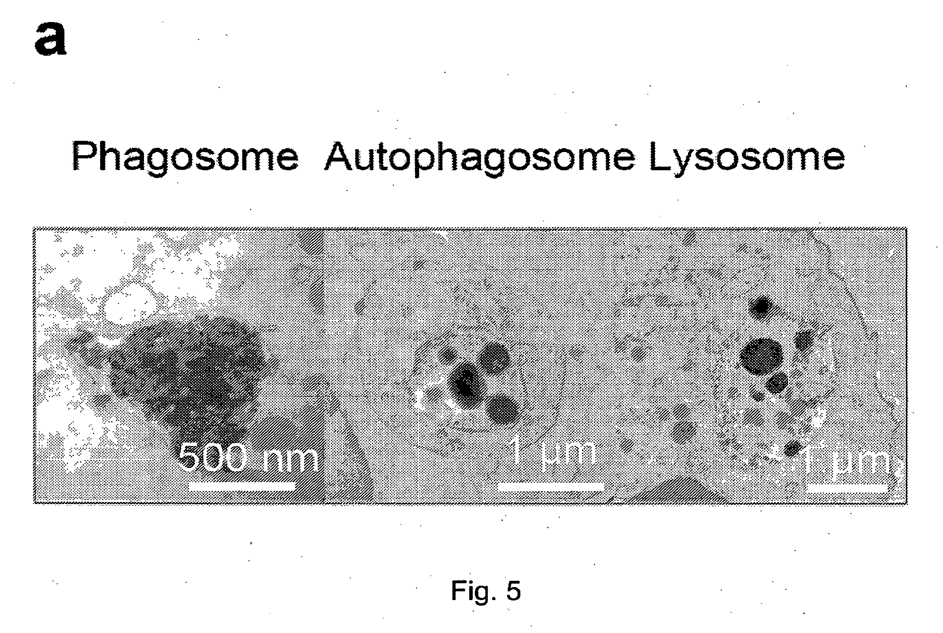

TEM images confirmed that α-Al₂O₃ NPs were found inside the endosomes/phagosomes, autophagosome, and autolysosomes (Fig. 5a). The internalized particles in DCs also were analyzed by an energy-dispersive X-ray (EDX) spectrometer. The EDX spectrum obtained confirmed the composition of the internalized nanoparticles as Al₂O₃.

As one of the major cellular pathways mediating degradation of proteins and organelles, autophagy is responsible for the MHC class II restricted presentation of endogenous antigens. Recent reports implicate autophagosomes of antigen donor cells for efficient cross-presentation, and that autophagy of pAPCs also regulates the cross-presentation of antigens derived from Herpes simplex virus (HSV) and Bacille Calmette-Guérin (BCG) for tuberculosis vaccine.

To test whether autophagy affects cross-presentation of α-Al₂O₃ NP-OVA, initiation of autophagy in DCs was inhibited by treatment with a phosphoinositide 3-kinase inhibitor. Specifically, 3-methyladenine (3-MA) (10 µM) or wortmannin (Calbiochem) (1.0 nM) was used to inhibit autophagy, and NH₄Cl (10 mM) was employed to block lysosome activity of DCs for 12 hours before loading antigens. For knockdown of the autophagy initiation gene Atg6/Beclin 1 or Atg12, siRNAs were prepared by *in vitro* transcription of T7 promoter tagged Beclin 1 and Atg12 cDNA templates with the easy siRNA kit (New England Biolabs). DCs were transfected with 0.3 µg Beclin 1 or Atg12 siRNA complexed with 1.4 µg INTERFERin^{™} (Polyplus) in a 24-well plate following the manufacturer's protocol. The knockdown of Beclin 1 or Atg12 was verified using Western blotting after incubation for 72 hours. Luciferase siRNA was prepared similarly and used as the control siRNA. To determine the autophagy level in DCs after pulsing with OVA or α-Al₂O₃ NP-OVA conjugates for 18 hours, the conversion of LC3 was determined by Western blotting assay analysis (Pierce). The DCs treated with NH₄Cl were used as the control. Brefeldin A (0.1µg/mL) was applied for 18 hours before loading antigen to inhibit Golgi-derived membrane, which effects the alternative, but not conventional autophagy.

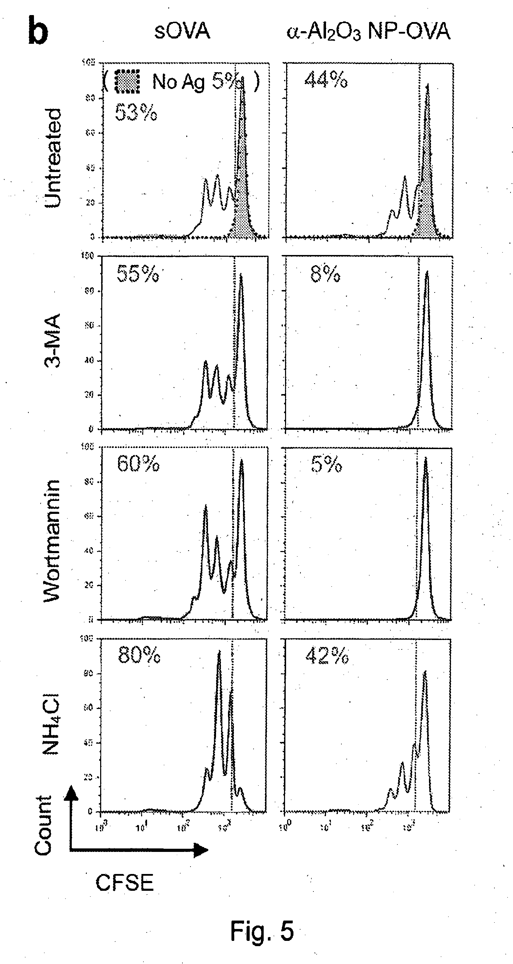

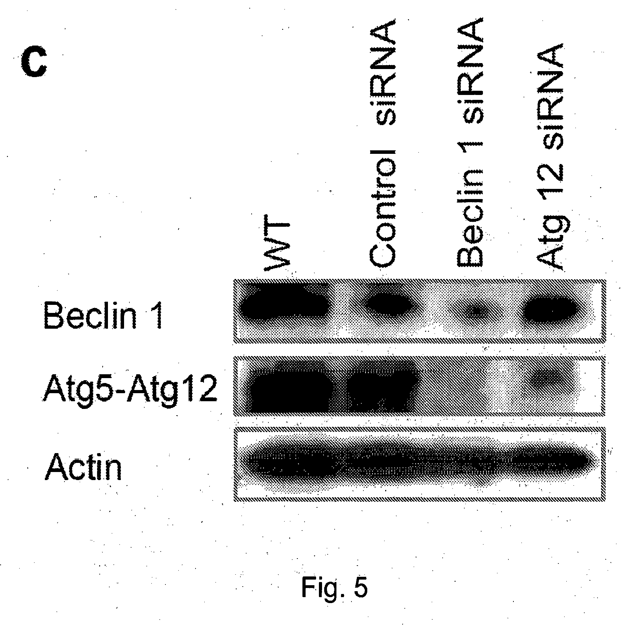

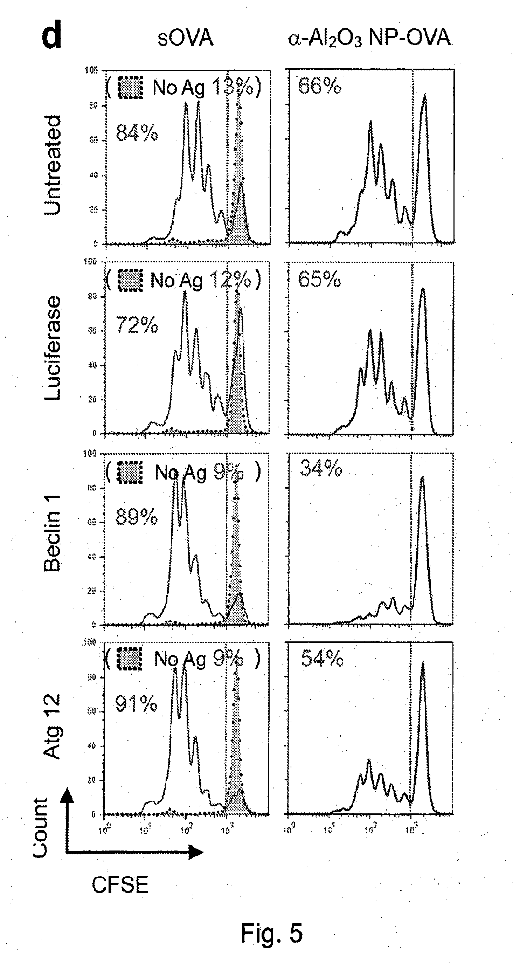

It was found that neither 3-MA or wortmannin reduced the cross-presentation of soluble OVA, but both inhibitors nearly abolished the cross-presentation of α-Al₂O₃ NP-OVA conjugates (Fig. 5b). Furthermore, knockdown of the autophagy initiation gene, Atg6/Beclin 1, in DCs blocked the cross-presentation of α-Al₂O₃ NP-OVA conjugates without affecting the cross-presentation of soluble OVA (Fig. 5c and 5d). These data suggest that the functional autophagy pathway is required for the cross-presentation of α-Al₂O₃ NP-OVA but not soluble OVA.

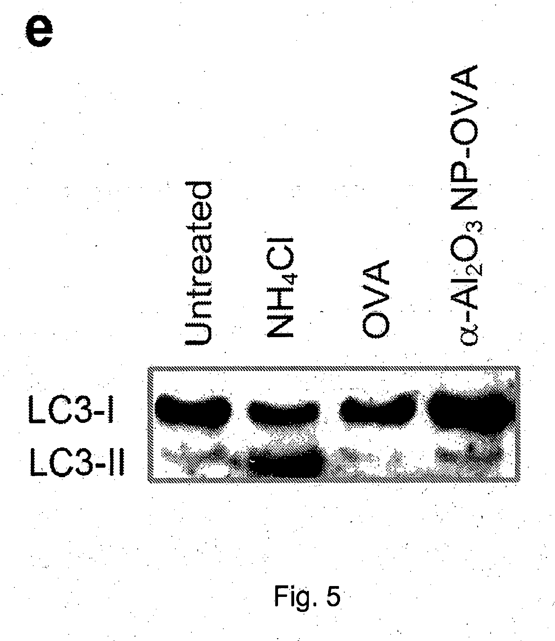

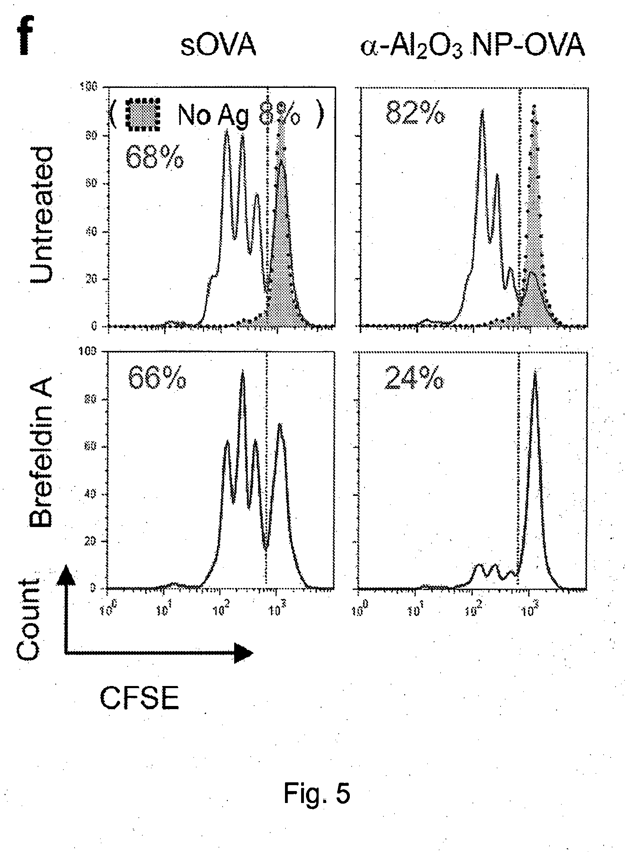

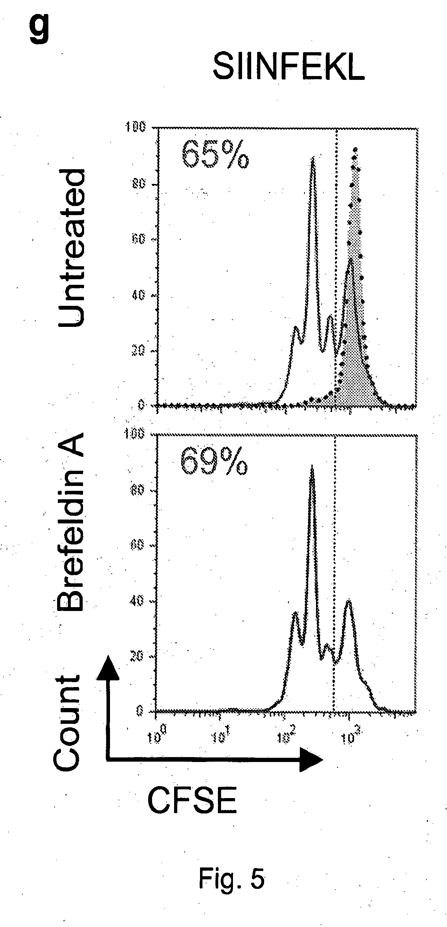

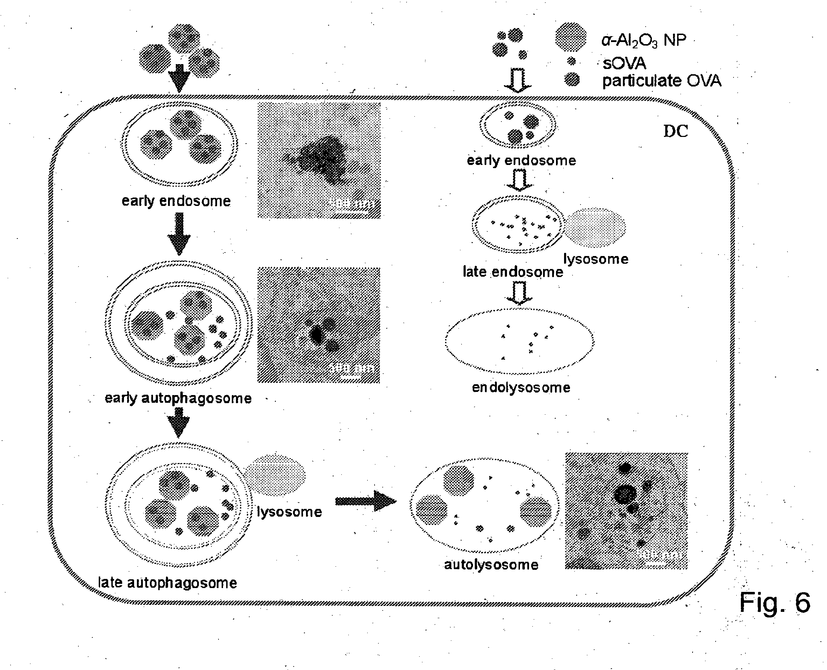

It also was found that compared to knockdown of Beclin 1, Atg 12 silencing less efficiently suppressed the cross-presentation of α-Al₂O₃ NP-OVA conjugates (Fig. 5d). In addition, the formation of autophagosomes by the uptake of α-Al₂O₃ NP-OVA conjugates did not significantly increase the production of LC3-II over the basal level of LC3-II in untreated DCs (Fig. 5e). Without wishing to be bound by any particular theory, it was hypothesized that α-Al₂O₃ NP-OVA conjugates might utilize an Atg12-independent (non-canonical) autophagy pathway for the efficient cross-presentation. Consistent with this hypothesis, it was found that cross-presentation of α-Al₂O₃ NP-OVA conjugates was blocked by brefeldin A, an inhibitor of ER-Golgi function that was used to distinguish the non-canonical from the canonical autophagy pathway (Figs. 5f and 5g). Because cross-presentation of exogenous antigens can be enhanced by blocking antigen degradation by lysosomes, it was hypothesized that inhibition of lysosomal activity could further increase the efficiency of cross-presenting OVA or α-Al₂O₃ NP-OVA conjugates. Treatment of DCs with NH₄Cl, a lysosomal blocker, was used to test this hypothesis, and it was found that the NH₄Cl treatment resulted in more efficient cross-presentation of OVA, without any effect on the cross-presentation of α-Al₂O₃ NP-OVA conjugates (Fig. 5b). These findings reveal that α-Al₂O₃ NP-mediated noncanonical autophagy diverted a significant amount of antigens into autophagosomes thus delaying acidification and degradation (Fig. 6).

Without wishing to be bound to any particular theory, it is believed, based upon the findings above, that α-Al₂O₃ NPs may divert antigens from direct endosome / lysosome degradation to indirect endosome / autophagosome lysosome pathway. Fig. 6 schematically compares the two pathways. Generally, soluble antigens such as OVA are internalized by macropinocytosis or receptor-mediated endocytosis and then degraded in an acidic environment after fusion of endosomes and lysosomes. Without wishing to be bound to any particular theory, it is believed that α-Al₂O₃ NP-carried antigens, in contrast, are delivered to autophagosomes after phagocytosis. By blocking the direct fusion of lysosomes and the endosomes encapsulating the α-Al₂O₃ NP-carried antigens, it is believed that the autophagic vacuoles maintain alkylination and reduce antigen degradation, resulting in an efficient cross-presentation of the antigen as chaperoned by the α-Al₂O₃ NPs.

### Example 6: Therapeutic efficacy of α-Al₂O₃ NP-antigen conjugate vaccine against established tumors

To examine whether α-Al₂O₃ NP-OVA conjugates could elicit an endogenous T cell response capable of eliminating established tumors, mice were injected with B16F10-OVA tumor cells and on day 7, tumor-bearing mice were injected subcutaneously with α-Al₂O₃ NP-OVA conjugates, α-Al₂O₃ NPs alone, or soluble OVA mixed with Alum (Rehydragel®). Intracellular IFN-γ staining was used to enumerate the frequency of the OVA-specific CD8⁺ T cells in spleens of naive or tumor-bearing mice 7 days post vaccination.

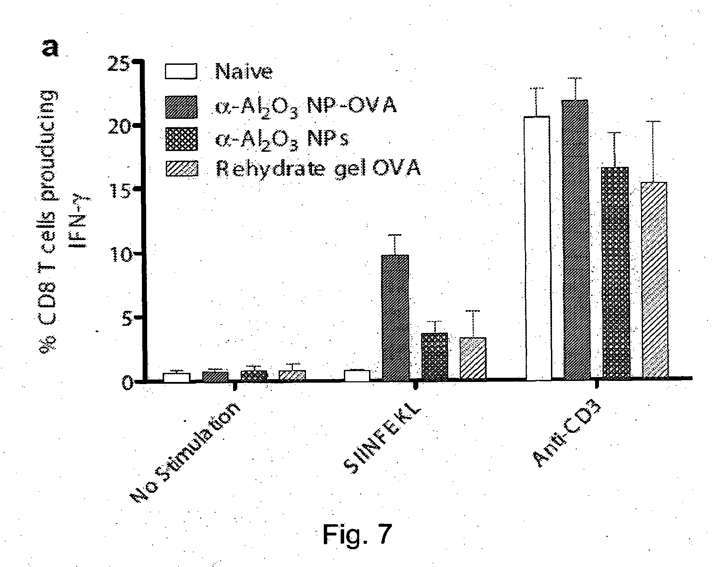

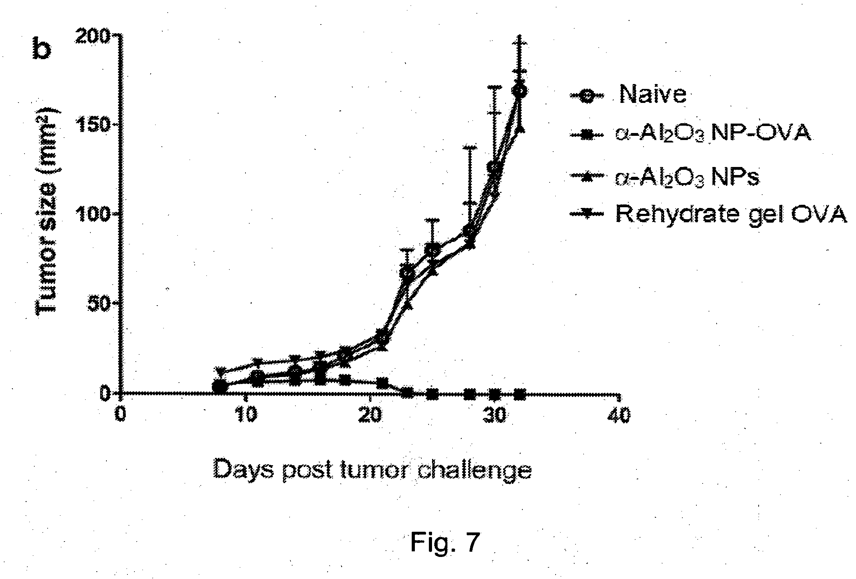

Fig. 7a shows the frequency of OVA-specific IFN-γ producing CD8⁺ T cells in spleens of mice bearing B16-OVA tumor cells after vaccination. As shown, mice vaccinated with α-Al₂O₃ NP-OVA conjugates exhibited a much higher level of OVA-specific T cells. Remarkably, the mice injected with α-Al₂O₃ NP-OVA conjugates completely rejected tumors and remained tumor free for more than 40 days, while all other groups succumbed to tumor burden (Fig. 7b).

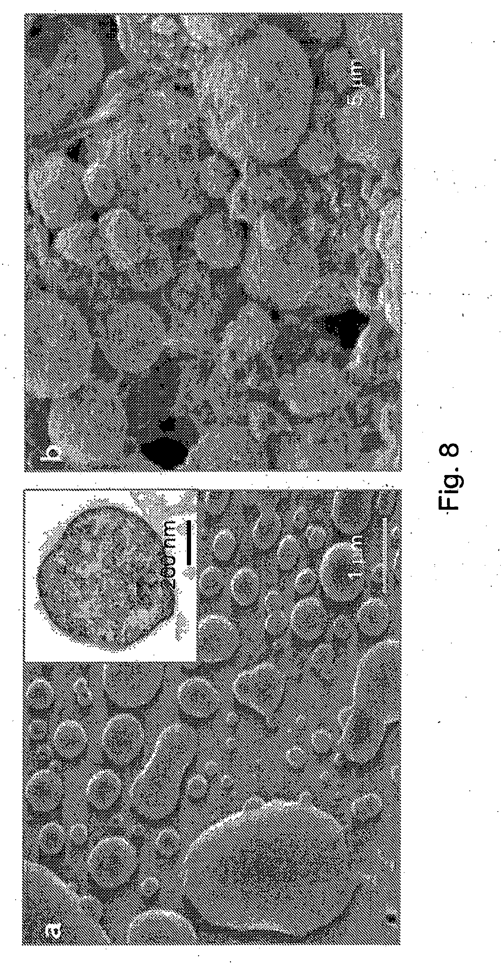

To further demonstrate the adjuvant activity of α-Al₂O₃ NPs to boost the T cell response to antigens presenting at limited amount in vaccines, studies were carried out to examine the ability of α-Al₂O₃ NPs (60 nm) to increase the cross-presentation of tumor-associated antigens. Specifically, tumor cell-derived autophagosomes (Fig. 8a) were used instead of whole tumor cells because conjugation of smaller autophagosomes (less than 1 µm) to α-Al₂O₃ NPs are easier than that of large tumor cells (more than 10 µm). Furthermore, compared to whole tumor cells, isolated autophagosomes are capable of cross-priming antigen-specific CD8⁺ T cells more efficiently. The tumor cells were engineered to express short-lived intracellular OVA so that successful cross-presentation could be assessed with Thy1.1⁺ OT-I CD8⁺ T-cell proliferation assay.

As described hereinabove, DRibbles are autophagosomes that encapsulate protein antigens (e.g., defective ribosomal products). DRibbles can be produced by contacting cells (e.g., tumor cells and cells infected with a target pathogen) with a proteasome inhibitor (including reversible proteasome inhibitors such as MG132 and irreversible proteasome inhibitors such as lactacystin and epoxomicin) and lysosomal blocker, and in some cases, also an autophagy inducer (for example, a cell-starving culture medium such as HBSS, and immunosuppresant drugs such as rapamycin). Their potency against tumor growth and their potential use as a cancer vaccine have been described in Li et al., Cancer Res., 68: 6889-6895 (2008) and International Publication No. WO 2007/016340.

For this study, isolated autophagosomes were prepared following the method reported in Li et al., Cancer Res., 68: 6889-6895 (2008). Briefly, tumor cells were cultured in RPMI complete media supplemented with 100 nM Bortizomib (proteasome inhibitor) and 20 mM NH₄Cl for 24 hours. Cells were collected and secreted autophagosomes (DRibbles) were dislodged from cells by vigorous washing of cells with solution D (150 mM NaCl and 5 mM EDTA). Autophagosomes (300-800 nm double membrane particles) were enriched by differential centrifugation method to remove large cell debris and small vesicles (<200 nm particles). The amount of proteins in the isolated autophagosomes was determined by BCA after lysis of autophagosomes with RIPA buffer. The isolated autophagosomes were conjugated to SFB modified α-Al₂O₃ NPs after modification with SANH as described above for OVA protein. The amounts of protein in the autophagosome or the α-Al₂O₃ NP-autophagosome suspensions were kept at 1 mg/ml, while the concentration of α-Al₂O₃ was around 1 mg/mL. The morphology and chemical composition of the α-Al₂O₃ NP-autophagosome conjugates were analyzed by an FEI Sirion field emission scanning electron microscope (SEM) equipped with an EDX spectrometer. Fig. 8b shows an SEM image of α-Al₂O₃ NP-autophagosome conjugates.

Melanoma model: Naive C57BL/6 mice were injected subcutaneously with 2 x 10⁵ B16-OVA cells. Seven days later, when tumors were palpable, mice were vaccinated by subcutaneous injection of α-Al₂O₃ NPs-OVA conjugates, NPs alone or OVA absorbed into Alum Rehydragel®) (eight mice per group). Three mice from each group were sacrificed and the frequency of OVA peptide-specific CD8⁺ T cells in spleens was determined by intracellular cytokine staining (ICS) after *in vitro* stimulation with the SIINFEKL peptide for 12 hours (BD bioscience). The growth of B16-OVA tumors in the remaining 5 mice were continuously monitored and measured.

Lung metastasis model: Eight-week-old male C57BL/6 mice were intravenously injected with lewis lung carcinoma cell lines (3LL) lung tumor cells (2 × 10⁵ per mouse) to establish experimental lung metastases. Treatment was started seven days later. The mice were vaccinated by subcutaneous injection of 3LL lung tumor cell-derived autophagosomes (100µg of proteins per mouse) or α-Al₂O₃ NP-autophagosome conjugates (100µg of proteins and 100µg of α-Al₂O₃ NPs per mouse). For some groups, 100 µg of anti-OX40 was co-administrated via intraperitoneal injection with vaccine. After 14 days, the lungs were harvested and fixed in Fekete's solution (100 mL of 70% alcohol, 10 mL of formalin, and 5 mL of glacial acetic acid). The number of metastases was counted in a double-blanked fashion.

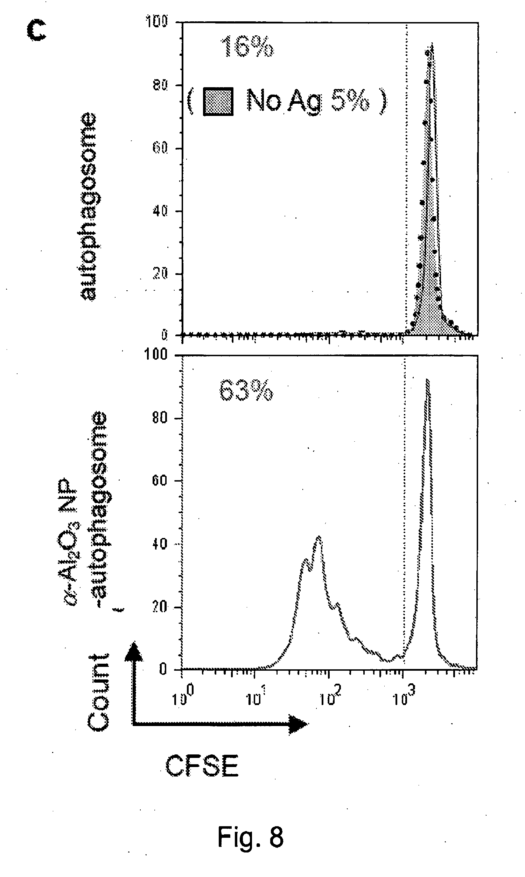

Compared to the DCs pulsed with naked autophagosomes, the DCs pulsed with α-Al₂O₃ NPs (60 nm) conjugated autophagosomes (Fig. 8b) were more efficient for cross-priming OT-I CD8⁺ T cells (Fig. 8c).

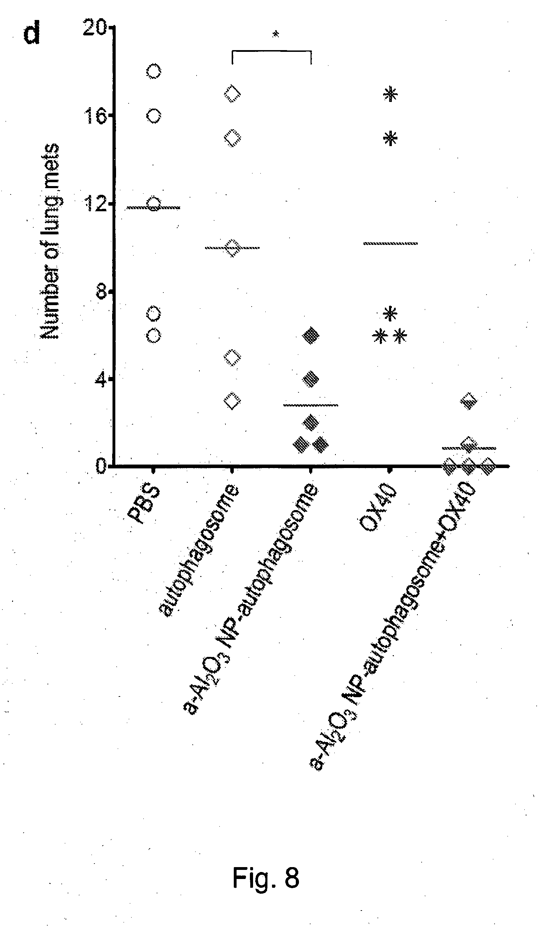

The therapeutic efficacy of autophagosomes and α-Al₂O₃ NP (60 nm)-autophagosome conjugates was evaluated in male C57BL/6 mice bearing experimental metastases 3LL lung tumors (Figure 8d). The employed autophagosomes were derived from unmodified 3LL tumor cells. The subcutaneous injection of α-Al₂O₃ NP-autophagosome conjugates but not naked autophagosomes significantly suppressed the formation of lung metastases as compared to PBS-treated control mice. Further improvement was achieved when mice were treated with both a vaccine containing α-Al₂O₃ NP-autophagosome conjugates and anti-OX40 antibody to promote the proliferation and survival of antigen-specific T cells. This combinational treatment led to zero metastases in 3 out of 5 mice, whereas no effect was observed in mice treated with OX40 antibody alone.

The above findings indicate that α-Al₂O₃ NPs can be used as effective new carriers for delivery of antigens to autophagosome-related cross-presentation pathway in pAPC to prime naïve antigen-specific T cells efficiently. When conjugated to tumor-derived autophagosome, α-Al₂O₃ NPs also are capable of boosting the antitumor efficacy of such tumor-derived autophagosomes containing complex unknown antigens.

## Claims

1. A nanoparticle-antigen conjugate capable of stimulating an immune response against a target antigen, the conjugate comprising:
an alumina nanoparticle having a diameter ranging from 5 nm to 150 nm; and
an autophagosome encapsulating defective ribosomal products (DRiPs) of the target antigen, wherein the autophagosome is covalently attached to the alumina nanoparticle.

2. The conjugate of claim 1, wherein the alumina nanoparticle is an α-Al₂O₃ nanoparticle having a diameter ranging from 10 nm to 120 nm.

3. The conjugate of claim 1 or 2, wherein the alumina nanoparticle has a diameter ranging from 10 nm to 80 nm.

4. The conjugate of any one of claims 1-3, wherein the autophagosome is produced by contacting cells comprising the target antigen with a proteasome inhibitor.

5. The conjugate of any one of claims 1-4; wherein the autophagosome is covalently attached to the alumina nanoparticle via bonds other than thioether bonds.

6. The conjugate of any one of claims 1-5, wherein the target antigen is a tumor-associated antigen, and the autophagosome is derived from a tumor cell.

7. The conjugate of any one of claims 1-5, wherein the target antigen is a pathogen-associated antigen, and the autophagosome is derived from a cell infected with a pathogen or a vector that expresses the pathogen-associated antigen.

8. The conjugate of any one of claims 1-5, wherein the target antigen is a virus-associated antigen, and the autophagosome is derived from a virus.

9. A nanoparticle-antigen conjugate capable of stimulating an immune response, the conjugate comprising:
an α-Al₂O₃ nanoparticle having a diameter ranging from 10 nm to 80 nm; and
an undefined antigen, wherein the undefined antigen is in the form of either a whole tumor cell or an autophagosome, and wherein the whole tumor cell or the autophagosome is covalently attached to the α-Al₂O₃ nanoparticle.

10. An immunogenic composition comprising a therapeutically effective amount of the conjugate of any one of claims 1-9 suspended in a pharmaceutically acceptable fluid carrier.

11. The immunogenic composition of claim 10, further comprising an anti-tumor chemotherapeutic agent, an immunostimulant, or combinations thereof.

12. The immunogenic composition of claim 10 or 11, wherein the immunogenic composition is a vaccine composition.

13. An immunogenic composition of any one of claims 10, 11 and 12 for use in stimulating an immune response against a target antigen in a subject.

14. The immunogenic composition of claim 13 for use according to claim 13, wherein the subject has a tumor.

15. The immunogenic composition of claim 13 or 14 for use according to claim 13, wherein the immune response is cell-mediated.

## Patentansprüche

1. Ein Nanopartikel-Antigen-Konjugat, das zur Stimulation einer Immunantwort gegen ein Zielantigen fähig ist, wobei das Konjugat Folgendes beinhaltet:
ein Aluminiumoxid-Nanopartikel mit einem Durchmesser im Bereich von 5 nm bis 150 nm; und
ein Autophagosom, das defekte ribosomale Produkte (*defective ribosomal products*, DRiPs) des Zielantigens einkapselt, wobei das Autophagosom kovalent an das Aluminiumoxid-Nanopartikel gebunden ist.

2. Konjugat gemäß Anspruch 1, wobei das Aluminiumoxid-Nanopartikel ein α-Al₂O₃-Nanopartikel mit einem Durchmesser im Bereich von 10 nm bis 120 nm ist.

3. Konjugat gemäß Anspruch 1 oder 2, wobei das Aluminiumoxid-Nanopartikel einen Durchmesser im Bereich von 10 nm bis 80 nm aufweist.

4. Konjugat gemäß einem der Ansprüche 1-3, wobei das Autophagosom durch das In-Kontakt-Bringen von Zellen, die das Zielantigen beinhalten, mit einem Proteasom-Inhibitor hergestellt wird.

5. Konjugat gemäß einem der Ansprüche 1-4, wobei das Autophagosom kovalent über andere Bindungen als Thioetherbindungen an das Aluminiumoxid-Nanopartikel gebunden ist.

6. Konjugat gemäß einem der Ansprüche 1-5, wobei das Zielantigen ein tumorassoziiertes Antigen ist und das Autophagosom aus einer Tumorzelle abgeleitet ist.

7. Konjugat gemäß einem der Ansprüche 1-5, wobei das Zielantigen ein pathogenassoziiertes Antigen ist und das Autophagosom aus einer Zelle abgeleitet ist, die mit einem Pathogen oder einem Vektor infiziert ist, das/der das pathogenassoziierte Antigen exprimiert.

8. Konjugat gemäß einem der Ansprüche 1-5, wobei das Zielantigen ein virusassoziiertes Antigen ist und das Autophagosom von einem Virus abgeleitet ist.

9. Ein Nanopartikel-Antigen-Konjugat, das zur Stimulation einer Immunantwort fähig ist, wobei das Konjugat Folgendes beinhaltet:
ein α-Al₂O₃-Nanopartikel mit einem Durchmesser im Bereich von 10 nm bis 80 nm;
und
ein undefiniertes Antigen, wobei das undefinierte Antigen in der Form von entweder einer ganzen Tumorzelle oder einem Autophagosom vorliegt, und wobei die ganze Tumorzelle oder das Autophagosom kovalent an das α-Al₂O₃-Nanopartikel gebunden ist.

10. Eine immunogene Zusammensetzung, die eine therapeutisch wirksame Menge des Konjugats gemäß einem der Ansprüche 1-9, suspendiert in einem pharmazeutisch akzeptablen fluiden Träger, beinhaltet.

11. Immunogene Zusammensetzung gemäß Anspruch 10, die ferner ein chemotherapeutisches Antitumormittel, ein Immunstimulans oder Kombinationen davon beinhaltet.

12. Immunogene Zusammensetzung gemäß Anspruch 10 oder 11, wobei die immunogene Zusammensetzung eine Impfstoff-Zusammensetzung ist.

13. Immunogene Zusammensetzung gemäß einem der Ansprüche 10, 11 und 12 zur Verwendung bei der Stimulation einer Immunantwort gegen ein Zielantigen in einem Probanden.

14. Immunogene Zusammensetzung gemäß Anspruch 13, wobei der Proband einen Tumor hat.

15. Immunogene Zusammensetzung gemäß Anspruch 13 oder 14, wobei die Immunantwort zellvermittelt ist.

## Revendications

1. Un conjugué nanoparticule-antigène capable de stimuler une réponse immunitaire contre un antigène cible, le conjugué comprenant :
une nanoparticule d'alumine ayant un diamètre allant de 5 nm à 150 nm ; et
un autophagosome encapsulant des produits ribosomiques défectueux (DRiP) de l'antigène cible, dans lequel l'autophagosome est attaché de manière covalente à la nanoparticule d'alumine.

2. Le conjugué de la revendication 1, dans lequel la nanoparticule d'alumine est une nanoparticule α-Al₂O₃ ayant un diamètre allant de 10 nm à 120 nm.

3. Le conjugué de la revendication 1 ou de la revendication 2, dans lequel la nanoparticule d'alumine a un diamètre allant de 10 nm à 80 nm.

4. Le conjugué de l'une quelconque des revendications 1 à 3, dans lequel l'autophagosome est produit en mettant en contact des cellules comprenant l'antigène cible avec un inhibiteur de protéasome.

5. Le conjugué de l'une quelconque des revendications 1 à 4, dans lequel l'autophagosome est attaché de manière covalente à la nanoparticule d'alumine via des liaisons autres que des liaisons thioéther.

6. Le conjugué de l'une quelconque des revendications 1 à 5, dans lequel l'antigène cible est un antigène associé à une tumeur, et l'autophagosome est dérivé d'une cellule tumorale.

7. Le conjugué de l'une quelconque des revendications 1 à 5, dans lequel l'antigène cible est un antigène associé à un agent pathogène, et l'autophagosome est dérivé d'une cellule infectée par un agent pathogène ou un vecteur qui exprime l'antigène associé à un agent pathogène.

8. Le conjugué de l'une quelconque des revendications 1 à 5, dans lequel l'antigène cible est un antigène associé à un virus, et l'autophagosome est dérivé d'un virus.

9. Un conjugué nanoparticule-antigène capable de stimuler une réponse immunitaire, le conjugué comprenant :
une nanoparticule α-Al₂O₃ ayant un diamètre allant de 10 nm à 80 nm ; et
un antigène non défini, dans lequel l'antigène non défini est sous la forme soit d'une cellule tumorale entière ou d'un autophagosome, et dans lequel la cellule tumorale entière ou l'autophagosome est attaché(e) de manière covalente à la nanoparticule α-Al₂O₃.

10. Une composition immunogène comprenant une quantité thérapeutiquement efficace du conjugué de l'une quelconque des revendications 1 à 9 en suspension dans un porteur liquide pharmaceutiquement acceptable.

11. La composition immunogène de la revendication 10, comprenant en outre un agent chimiothérapique anti-tumoral, un immunostimulant, ou des combinaisons de ceux-ci.

12. La composition immunogène de la revendication 10 ou de la revendication 11, dans laquelle la composition immunogène est une composition de vaccin.

13. Une composition immunogène de l'une quelconque des revendications 10, 11 et 12 pour une utilisation dans la stimulation d'une réponse immunitaire contre une cible antigène chez un sujet.

14. La composition immunogène de la revendication 13 pour une utilisation selon la revendication 13, dans laquelle le sujet a une tumeur.

15. La composition immunogène de la revendication 13 ou de la revendication 14 pour une utilisation selon la revendication 13, dans laquelle la réponse immunitaire est à médiation cellulaire.
